# EUROPÄISCHE PATENTSCHRIFT

(11) **EP 0 888 353 B1**
(45) Veröffentlichungstag und Bekanntmachung des Hinweises auf die Patenterteilung: **09.07.2003**
(21) Anmeldenummer: 97914189.2
(22) Anmeldetag: 05.03.1997
(51) Int. Cl.: C07D 487/04, A61K 31/505

(54) **N-7 HETEROCYCLYL-PYRROLO[ 2,3-d]PYRIMIDINE UND IHRE VERWENDUNG**
N-7-HETEROCYCLYL PYRROLO [2,3-d]PYRIMIDINES AND THEIR USE
N-7-HETEROCYCLYL-PYRROLO[ 2,3-d]PYRIMIDINES ET LEUR UTILISATION

(30) Priorität: 15.03.1996 CH 69496
(43) Veröffentlichungstag der Anmeldung: 07.01.1999
(73) Patentinhaber: Novartis AG, 4056 Basel (CH); Novartis Pharma GmbH, 1230 Wien (AT)
(72) Erfinder: ALTMANN, Eva, CH-4153 Reinach (CH)
(86) Internationale Anmeldenummer: EP9701095
(87) Internationale Veröffentlichungsnummer: WO97034895

(56) Entgegenhaltungen:
- EP-A- 0 496 617

## Beschreibung

Die Erfindung betrifft N-7-Heterocyclyl-pyrrolo[2,3-d]pyrimidin Derivate, welche als Protein Tyrosin Kinase-Inhibitoren wirken.

EP 0 496 617 A1 (Gensia Pharmaceuticals Inc.) beschreibt Nukleosidanaloge, die eine 5'modifizierte Ribose enhalten, welche an eine substituierte Pyrrolo[2,3-d]pyrimidino bzw. Pyrazolo[3,4-d]pyrimidino Base gebunden ist. Diese Nukleosidanaloge wirken als Inhibitoren der Adenosin Kinasen und Können Klinish eingesetzt werden, um Krankheiten zu behandeln, bei denen eine erhöhte lokalisierte Adenosin Konzentration von Vorteil ist.

Die Erfindung betrifft Verbindungen der Formel I, 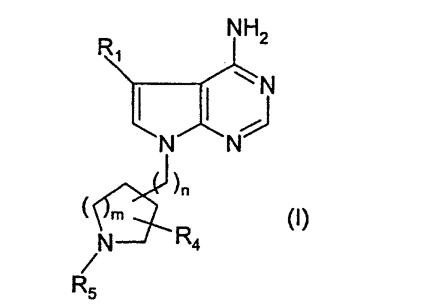 worin
R₁ für Phenyl steht, das unsubstituiert oder durch einen, zwei oder drei Substituenten aus der Gruppe bestehend aus Niederalkyl, Hydroxyniederalkyl, Phenyl, Niederalkoxy, Phenyl-niederalkoxy, C₁-C₃-Alkylendioxy, Cyano Hydroxy und Halogen substituiert ist;
R₄ für Wasserstoff, unsubstituiertes oder substituiertes Niederalkyl, Niederalkoxy, Niederalkylenoxyniederalkyl, Niederalkoxycarbonyl, N-Niederalkylaminocarbonyl, N,N-Diniederalkylaminocarbonyl, wobei die Substituenten aus der Gruppe bestehend aus Halogen, Hydroxy, Niederalkoxy, Trifluromethyl, Morpholin-4-yl, Amino N-Niederalkylamino oder N,N-Diniederalkylamino ausgewählt sind;
R₅ für Wasserstoff oder unsubstitiertes oder substituiertes Niederalkyl, Niederalkylcarbonyl oder Niederalk oxycarbonyl wobei die Substituenten aus der Gruppe bestehend aus Halogen, Hydroxy, Niederalkoxy, Trifluromethyl, Morpholin-4-yl, Amino N-Niederalkylamino oder N,N-Diniederalkylamino ausgewählt sind;
m für 1 oder 2 steht;
n für 0 oder 1 steht; und :

### Beispiel 26: (2R/4S)-[4-Amino-5-(4-hydroxy-phenyl)-pyrrolo[2,3-d]pyrimidin-7-yl]-1-(3,3-dimethyl-butyryl)-pyrrolidin-2-carbonsäure-ethylester

### Beispiel 47: (2S/4R)-2-(4-Amino-5-phenyl-pyrrolo[2,3-d]pyrimidin-7-ylmethyl)-4-hydroxy-pyrrolidin-1-carbonsäure-tert.-butylester

### Beispiel 48: (3R/5S)-5-(4-Amino-5-phenyl-pyrrolo[2,3-d]pyrimidin-7-ylmethyl)-pyrrolidin-3-ol

### Beipiel 49: (2R/4R)-2-(4-Amino-5-phenyl-pyrrolo[2,3-d]pyrimidin-7-ylmethyl)-4-hydroxy-pyrrolidin-1-carbonsäure-tert.-butylester

### Beispiel 50: (3R/5R)-5-(4-Amino-5-phenyl-pyrrolo[2,3-d]pyrimidin-7-ylmethyl)-pyrrolidin-3-ol

### Beispiel 81: 7-[1-(2-Imidazol-1-yl-ethyl)-pyrrolidin-3-yl]-5-phenyl-7H-pyrrolo[2,3-d]pyrimidin-4-yl-amin

### Beispiel 84: 4-(4-Amino-7-{1-[2-(2-hydroxy-ethylamino)-ethyl]-pyrrolidin-3-yl}-7H-pyrrolo-[2,3-d]pyrimidin-5-yl)-phenol

### Beispiel 85: 3-(4-Amino-7-{1-[2-(2-hydroxy-ethylamino)-ethyl]-pyrrolidin-3-yl}-7H-pyrrolo-[2,3-d]pyrimidin-5-yl)-phenol

### Beispiel 88: 4-{4-Amino-7-[1-(2-imidazol-1-yl-ethyl)-pyrrolidin-3-yl]-7H-pyrrolo[2,3-d]pyrimidin-5-yl}-phenol

### Beispiel 89: 3-{4-Amino-7-[1-(2-imidazol-1-yl-ethyl)-pyrrolidin-3-yl]-7H-pyrrolo[2,3-d]pyrimidin-5-yl}-phenol

### Beispiel 96: {3-[4-Amino-5-(3-benzyloxy-phenyl)-pyrrolo[2,3-d]pyrimidin-7-yl]-pyrrolidin-1-yl}essigsäure-methylester

### Beispiel 97: {3-[4-Amino-5-(3-hydroxy-phenyl)-pyrrolo[2,3-d]pyrimidin-7-yl]-pyrrolidin-1-yl}essigsäure-methylester

### Beispiel 98: 2-{3-[4-Amino-5-(3-hydroxy-phenyl)-pyrrolo[2,3-d]pyrimidin-7-yl]-pyrrolidin-1-yl}-N,N-dimethyl-acetamid

### Beispiel 99: 2-{3-f4-Amino-5-(3-hydroxy-phenyl)-pyrrolo[2,3-d]pyrimidin-7-yl]-pyrrolidin-1-yl}acetamid

### Beispiel 100: 2-(2-{3-[4-Amino-5-(4-methoxy-phenyl)-pyrrolo[2,3-d]pyrimidin-7-yl]-pyrrolidin-1-yl}-ethylamino)-ethanol

### Beispiel 101: 2-(2-{3-[4-Amino-5-(3-methoxy-phenyl)-pyrrolo[2,3-d]pyrimidin-7-yl]-pyrrolidin-1-yl}-ethylamino)-ethanol

### Beispiel 104: 7-[1-(2-Imidazol-1-yl-ethyl)-pyrrolidin-3-yl]-5-(4-methoxy-phenyl)-7H-pyrrolo-[2,3-d]pyrimidin-4-yl-amin

### Beispiel 105: 7-[1-(2-lmidazol-1-yl-ethvl)-pyrrolidin-3-yl]-5-(3-methoxy-phenyl)-7H-pyrrolo-[2,3-d]pyrimidin-4-yl-amin

### Beispiel 108: {3-[4-Amino-5-(3-methoxy-phenyl)-pyrrolo[2,3-d]pyrimidin-7-yl]-pyrrolidin-1-yl}essigsäure-methylester

### Beispiel 129: 7-{1-[2-(2-Amino-ethoxy)-ethyl]-piperidin-4-yl}-5-phenyl-7H-pyrrolo[2,3-d]pyrimidin-4-yl-amin

### Beispiel 130: 7-[1-(2-Imidazol-1-yl-ethyl)-piperidin-4-yl]-5-phenyl-7H-pyrrolo[2,3-d]pyrimidin-4-yl-amin

### Beispiel 135: 7-{1-[2-(2-Amino-ethoxy)-ethyl]-piperidin-4-yl}-5-(4-methoxy-phenyl)-7Hpyrrolo[2,3-d]pyrimidin-4-yl-amin

### Beispiel 136: 7-{1-[2-(2-Amino-ethoxy)-ethyl]-piperidin-4-yl}-5-(3-methoxy-phenyl)-7Hpyrrolo[2,3-d]pyrimidin-4-yl-amin

### Beispiel 137: 7-[1-(2-Imidazol-1-yl-ethyl)-piperidin-4-yl]-5-(4-methoxy-phenyl)-7H-pyrrolo-[2,3-d]pyrimidn-4-yl-amin

### Beispiel 138: 7-[1-(2-Imidazol-1-yl-ethyl)-piperidin-4-yl]-5-(3-methoxy-phenyl)-7H-pyrrolo-[2,3-d]pyrimidn-4-yl-amin

### Beispiel 144: {4-[4-Amino-5-(3-methoxy-phenyl)-pyrrolo[2,3-d]pyrimidin-7-yl]-piperidin-1-yl}essigsäure-methylester

### Beispiel 145: 2-{4-[4-Amino-5-(3-methoxy-phenyl)-pyrrolo[2,3-d]pyrimidin-7-yl]-piperidin-1-yl}acetamid

### Beispiel 146: 2-{4-[4-Amino-5-(3-methoxy-phenyl)-pyrrolo[2,3-d]pyrimidin-7-yl]-piperidin-1-yl}-N,N-dimethyl-acetamid

### Beispiel 147: {4-[4-Amino-5-(3-methoxy-phenyl)-pyrrolo[2,3-d]pyrimidin-7-yl]-piperidin-1-yl}essigsäure

### Beispiel 152: 4-(4-Amino-7-{1-[2-(2-amino-ethoxy)-ethyl]-piperidin-4-yl}-7H-pyrrolo[2,3-d]pyrimidin-5-yl)-phenol

### Beispiel 153: 3-(4-Amino-7-{1-[2-(2-amino-ethoxy)-ethyl]-piperidin-4-yl}-7H-pyrrolo[2,3-d]-pyrimidin-5-yl)-phenol

### Beispiel 156: 4-{4-Amino-7-[1-(2-imidazol-1-yl-ethyl)-piperidin-4-yl]-7H-pyrrolo[2,3-d]pyrimidin-5-yl}-phenol

### Beispiel 157: 3-{4-Amino-7-[1-(2-imidazol-1-yl-ethyl)-piperidin-4-yl]-7H-pyrrolo[2,3-d]pyrimidin-5-yl}-phenol

### Beispiel 158: {4-[4-Amino-5-(3-hydroxy-phenyl)-pyrrolo[2,3-d]pyrimidin-7-yl]-piperidin-1-yl}essigsäure-methylester

### Beispiel 159: 2-{4-[4-Amino-5-(3-hydroxy-phenyl)-pyrrolo[2,3-d]pyrimidin-7-yl]-piperidin-1-yl}-N,N-dimethyl-acetamid

### Beispiel 160: 2-{4-[4-Amino-5-(3-hydroxy-phenyl)-pyrrolo[2,3-d]pyrimidin-7-yl]-piperidin-1-yl}acetamid

### Beispiel 161: {4-[4-Amino-5-(3-hydroxy-phenyl)-pyrrolo[2,3-d]pyrimidin-7-yl]-piperidin-1-yl}essigsäure

"Nieder" bezeichnet einen Rest bis und mit 7 Kohlenstoffatomen; und Salze davon; insbesondere pharmazeutisch verträgliche Salze; Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7 und insbesondere bis und mit 6 Kohlenstoffatomen.

Niederalkyl ist z. B. *n*-Propyl, Isopropyl, *n*-Butyl, Isobutyl, *sek*-Butyl, *tert*-Butyl, *n*-Pentyl, Neopentyl, *n*-Hexyl oder *n*-Heptyl, vorzugsweise Ethyl oder Methyl.

Niederalkylen ist z. B. Methylen, Ethylen, Propylen, vorzugsweise Methylen oder Ethylen.

Alkyl und Alkylen sind geradkettig oder verzweigt. Für sich, wie beispielsweise Niederalkyl, oder als Bestandteil anderer Gruppen, wie z. B. Niederalkoxy, Niederalkylcarbonyl, Niederalkoxycarbonyl, Niederalkylaminocarbonyl, Diniederalkylaminocarbonyl, Niederalkoxyniederalkylenyl, können sie unsubstituiert oder substituiert sein, mit Halogen, Hydroxy, Niederalkoxy, Trifluoromethyl, oder Morpholin-4-yl, vorzugsweise sind sie unsubstituiert oder mit Hydroxy oder Amino, N-Niederalkylamino oder N,N-Diniederalkylamino substituiert.

Halogen ist z. B. Chlor, Brom oder Fluor, kann aber auch lod bedeuten.

Niederalkoxy ist z. B. *n*-Propoxy, Isopropoxy, *n*-Butoxy, Isobutoxy, *sek*-Butoxy, *tert*-Butoxy, *n*-Amyloxy, Isoamyloxy, vorzugsweise Methoxy und Ethoxy.

Niederalkoxycarbonyl bezeichnet den Rest Niederalkyl-O-C(O)- und ist z. B. *n*-Propoxycarbonyl, Isopropoxycarbonyl, *n*-Butoxycarbonyl, Isobutoxycarbonyl, *sek*-Butoxycarbonyl, *tert*-Butoxycarbonyl, *n*-Amyloxycarbonyl, Isoamyloxycarbonyl, vorzugsweise Methoxycarbonyl und Ethoxycarbonyl.

Niederalkylamino ist beispielsweise *n*-Propylamino, *n*-Butylamino, *i*-Propylamino, *i*-Butylamino, vorzugsweise Methylamino und Ethylamino.

Diniederalkylamino ist beispielsweise Dimethylamino, Diethylamino, Di-*n*-Propylamino, *n*-Butylamino, Di-*n*-butylamino, n-Propyl-*n*-butylamino, vorzugsweise Dimethylamino, Diethylamino und Methylethylamino.

N-Niederalkylaminocarbonyl ist z. B. N-Methylcarbamoyl, N-Ethylcarbamoyl, N-*n*-Propylcarbamoyl, N-lsopropylcarbamoyl, vorzugsweise N-Methylcarbamoyl und N-Ethylcarbamoyl.

N,N-Diniederalkylaminocarbonyl ist beispielsweise N,N-Dimethylcarbamoyl, N,N-Diethylcarbamoyl, N-Methyl-N-ethylcarbamoyl, N,N-Di-*n*-Propylcarbamoyl, N-Methyl-N-isopropylcarbamoyl, vorzugsweise N,N-Dimethylcarbamoyl, N,N-Diethylcarbamoyl und N-Methyl-N-ethylcarbamoyl.

Die Verbindungen I und ihre Salze können in Form eines der möglichen Isomeren, beispielsweise Stereoisomere, Diastereoisomere oder Tautomere, oder als Gemisch derselben vorliegen. Dabei sind als reine Isomere z. B. reine Enantiomere, reine Diastereoisomere oder, gegebenenfalls, reine Tautomere erhältlich. Entsprechend können als Isomerengemische z. B. Racemate oder Diastereoisomerengemische vorliegen.

Salze von Verbindungen der Formel I sind insbesondere pharmazeutisch verwendbare Salze, in erster Linie Säureadditionssalze mit geeigneten Mineralsäuren, wie Halogenwasserstoffsäuren, Schwefelsäure oder Phosphorsäure, z. B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z. B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z. B. Acetate, Oxalate, Malonate, Maleinate, Fumarate, Tartrate oder Citronate.

Sofern die Verbindungen der Formel I eine saure Gruppe enthalten, sind auch entsprechende Salze mit Basen möglich, z. B. entsprechende Alkalimetall- oder Erdalkalimetallsalze, z. B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z. B. Mono-, Di- oder Trihydroxyniederalkylaminen, Hydroxyniederalkyl-niederalkyl-aminen oder Polyhydroxyniederalkylaminen. Cyclische Amine sind z. B. Morpholin, Thiomorpholin, Piperidin oder Pyrrolidin. Als Mononiederalkylamine kommen beispielsweise Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z. B. Mono-, Di- und Triethanolamin; Hydroxyniederalkyl-niederalkyl-amine sind z. B. N,N-Dimethylamino- und N,N-Diethylamino-ethanol. Verbindungen der Formel I mit einer sauren Gruppe, z. B. Carboxy, und einer basischen Gruppe, z. B. Amino, können z. B. auch in Form von inneren Salzen, d.h. in zwitterionischer Form vorliegen, oder es kann ein Teil des Moleküls als inneres Salz, und ein anderer Teil als normales Salz vorliegen. Umfasst sind ferner für pharmazeutische Verwendungen ungeeignete Salze, da diese beispielsweise für die Isolierung bzw. Reinigung von freien Verbindungen I sowie von deren pharmazeutisch verwendbaren Salzen verwendet werden können.

Die Verbindungen der Formel I haben wertvolle pharmakologische Eigenschaften. Insbesondere hemmen sie die Aktivität der Protein-Tyrosin-Kinase pp60^{C-SrC} in Konzentrationen zwischen ca. 0.001 und ca. 10 µM [Testbeschreibung: K. Farley et al., *Anal. Biochem.* **203** (1992) 151-157; dabei wird gereinigtes Enzym - wie in N. B. Lydon et al., *Biochem. J.* **287** (1992) 985-993 beschrieben - verwendet].

Es ist bekannt, dass sowohl eine gezielte Veränderung des c-src Gens, die zur Elimination von c-src führt, als auch eine Hemmung der Aktivität der Protein-Tyrosin-Kinase pp60^{c-src} die Fähigkeit von Osteoklasten zur Knochenresorption beeinflusst [Elimination von c-src durch Genmanipulation: siehe z. B. P. Soriano et al., *Cell* **64** (1991) 693-702; Hemmung der Aktivität der Protein-Tyrosin-Kinase pp60^{c-src}: siehe z. B. B.F. Boyce et al., *J. Clin. Invest.* **90** (1992) 1622-1627; T. Yoneda et al., J. Clin. Invest. 91 (1993) 2791-2795].

Aufgrund ihrer Hemmwirkung auf die Protein-Tyrosin-Kinase pp60^{c-src} sind die Verbindungen der Formel I daher in der Lage, die Fähigkeit der Osteoklasten zur Knochenresorption zu hemmen. Dies kann z. B. im sogenannten "bone slice assay" an kortikalen Knochenplättchen von Rindern mit Rattenosteoklasten in Konzentrationen zwischen ca. 0.001 und ca. 10 µM nachgewiesen werden [Der "bone slice assay" ist z. B. beschrieben in *Biochem*. *Biophys. Res. Comm.* **188** (1992) 1097-1103]. Die Verbindungen der Formel I hemmen dabei die Bildung von charakteristischen Resorptionslöchern auf Knochenplättchen in vitro.

In vivo kann die Wirksamkeit von Verbindungen der Formel I z. B. im Hock-Modell an der Ratte nachgewiesen werden. In diesem Test hemmen die Verbindungen der Formel I - bei einmaliger Applikation *per os* pro Tag in Konzentrationen zwischen ca. 1 und ca. 100 mg/kg Körpergewicht - während 3-4 Wochen vollständig oder zumindest partiell den Knochenverlust, der durch Ovariektomie in Ratten erzeugt wird [Das "Hock-Modell" ist z. B. beschrieben in *Metab. Bone Dis*. **5** (1984) 177-181].

Die in vivo Wirksamkeit von Verbindungen der Formel I kann auch z. B. via Calcium-Metabolismus an intakten Ratten nachgewiesen werden. Dabei wird nach i.v. Injektion der Testsubstanz innerhalb 1-4 Stunden eine akute Hypokalzämie induziert, die durch Bestimmung der Calcium-Konzentration im Blutplasma nachgewiesen wird. Die Beobachtung einer akuten Hypokalzämie kann als indirekter Nachweis für eine Hemmung der Knochenresorption durch die Testsubstanz interpretiert werden.

Die Verbindungen der Formel I sind daher sehr gut geeignet zur Behandlung von Krankheiten, die auf eine Hemmung der Aktivität der Protein-Tyrosin-Kinase pp60^{c-src} ansprechen. Zu nennen ist hier insbesondere die Osteoporose, femer auch andere Krankheiten, in deren Verlauf die Knochenresorption durch Osteoklasten eine Rolle spielt, wie z. B. die Tumorinduzierte Hyperkalzämie, Paget's Disease oder die Behandlung von Knochenmetastasen, des weiteren auch inflammatorische Prozesse in Gelenken und Knochen sowie degenerative Prozesse in Knorpelgeweben. Darüber hinaus sind die Verbindungen der Formel I nützlich zur Behandlung von benignen oder malignen Tumoren, die auf eine Hemmung der Protein-Tyrosin-Kinase pp60^{c-src} ansprechen, wie z. B. Brustkrebs (Mammakarzinom) oder Darmkrebs (Colonkarzinom). Sie sind in der Lage, Tumorregression zu bewirken und Tumormetastasierung und das Wachstum von Mikrometastasen zu verhindern. Die Verbindungen der Formel I sind femer nützlich bei der Behandlung kardiovaskulärer Erkrankungen, wie z. B. Thrombose.

Die Verbindungen der Formel I hemmen ferner auch die Aktivität von anderen Nicht-Rezeptor-Protein-Tyrosin-Kinasen, wie z. B. (a) anderen Mitgliedern der src-Familie, z. B. Ick und fyn, (b) Abl-Kinase sowie (c) ZAP70-Kinase. Darüber hinaus hemmen die Verbindungen der Formel I auch die Aktivität von Rezeptor-Protein-Tyrosin-Kinasen, wie z. B. (a) der EGF-Familie, z. B. den EGF-Rezeptor, c-erbB2, c-erbB3 und c-erbB4, und (b) der PDGF-Familie, z. B. den PDGF-Rezeptor, CSF-1, Kit, VEGF und FGF. Aufgrund dieser Wirkungen können die Verbindungen der Formel I auch zur Immunomodulation und zur Behandlung von Erkrankungen des Immunsystems eingesetzt werden, z. B. bei Entzündungen oder Organtransplantationen. Weiterhin sind sie geeignet zur Behandlung (hyper)proliferativer Erkrankungen, wie z. B. Psoriasis, Tumoren, Karzinomen und Leukämien, sowie bei Fibrose und Restenose. Auch bei der Behandlung von Erkrankungen des zentralen oder peripheren Nervensystems können die Verbindungen der Formel I angewendet werden, soweit eine Signalübertragung durch mindestens eine Protein-Tyrosin-Kinase involviert ist.

Die Erfindung betrifft insbesondere die in den Beispielen beschriebenen spezifischen Verbindungen und Salze davon.

Die Verbindungen der Formel I können in an sich bekannter Weise hergestellt werden, indem man z. B.
(a) eine Verbindung der Formel II 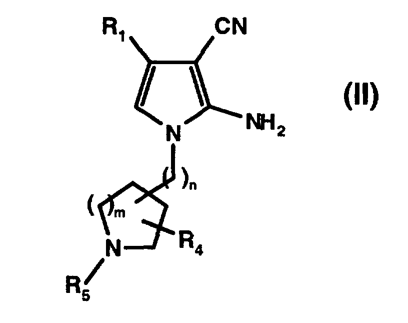 einer Ringschlussreaktion unter Aufbau des Pyrimidinrings unterwirft, oder
(b) eine Verbindung der Formel III 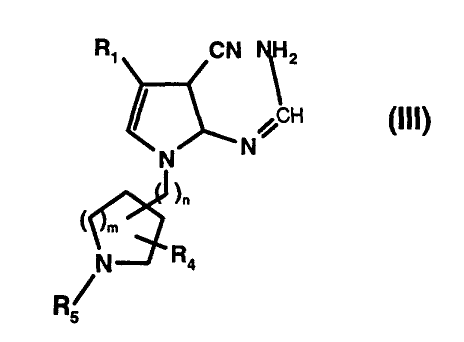 einer Ringschlussreaktion unter Aufbau des Pyrimidinrings unterwirft, oder
(c) eine Verbindung der Formel IV 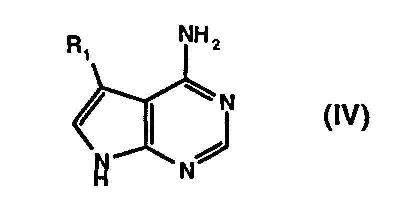 mit einer Verbindung der Formel V 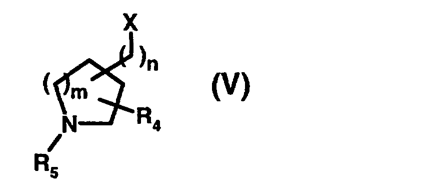 worin X für eine Abgangsgruppe steht, umsetzt, oder
(d) eine Verbindung der Formel VI 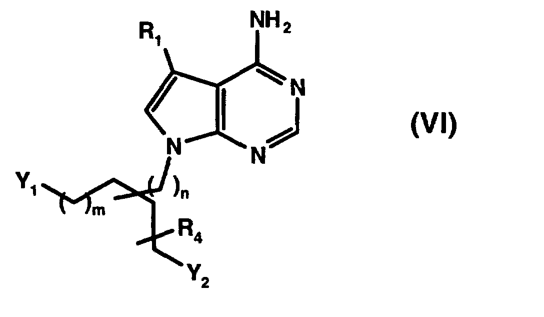
worin Y₁ und Y₂ für geeignete Abgangsgruppen, beispielsweise Halogen, insbesondere Chlor, stehen, mit einer Verbindung der Formel VII

**R**_{**5**}**―NH**_{**2**} **(VII)**

umsetzt,
und, wenn erwünscht, eine Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

In der folgenden näheren Beschreibung der Verfahren haben die Symbole R₁-R₅ jeweils die unter Formel I angegebene Bedeutung, sofern nichts anderes angegeben ist.

Verfahren (a): Die Umsetzung gemäss dem Verfahren (a) entspricht der an sich bekannten Cyclisierung von 2-Amino-3-cyano-pyrrolen zu 4-Amino-pyrrolo[2,3-d]pyrimidinen (s. z. B. H. Pichler et al., *Liebigs Ann. Chem*. **1986**, 1485-1505). Als Cyclisierungsreagenzien kommen z. B. (1) Formamid oder (2) 1. Orthoameisensäuretrialkylester/2. Ammoniak in Frage. Die Cyclisierung der Verbindungen der Formel II mit Formamid wird vorzugsweise bei erhöhter Temperatur, z. B. 160 °C, und vorteilhaft unter Zugabe von wenig Dimethylformamid und Ameisensäure durchgeführt. Die Umsetzung der Verbindungen der Formel II mit Orthoameisensäuretrialkylester zu den entsprechenden, intermediär entstehenden Alkoxyformimidaten erfolgt normalerweise bei weniger stark erhöhten Temperaturen, z. B. 80-120 °C. Die Cyclisierung der letzteren mit Ammoniak wird dann in der Regel wieder bei höheren Temperaturen, z. B. bei 130 °C im Autoklav, durchgeführt.

Die Verbindungen der Formel II werden vorzugsweise unter Anwendung einer der bekannten Pyrrolsynthesen hergestellt. Man erhält sie z. B. durch Umsetzung einer Verbindung der Formel IIa 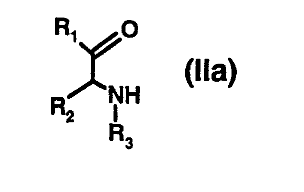 mit Malonsäuredinitril, vorzugsweise in Gegenwart einer Base, z. B. Natrium-ethoxid/Ethanol.

Die Verbindungen der Formel lla ihrerseits lassen sich z. B. durch Umsetzung einer Verbindung R₁-C(=O)-CH(-R₂)-Hal [Hal = Halogen], also z. B. Phenacylbromid oder -chlorid, mit einer Verbindung H₂N-R₃, z. B. Anilin, herstellen, vorzugsweise in Gegenwart einer Base, z. B. Natriumcarbonat/Ethanol oder Triethylamin/Toluol.

Verfahren (b): Der Ringschluss zum entsprechenden 4-Amino-pyrrolo[2,3-d]pyrimidin erfolgt z. B. in Gegenwart von geeigneten Basen, z. B. Natriumethoxid/Ethanol, vorzugsweise bei erhöhter Temperatur, z. B. 80 °C [s. z. B. E.C. Taylor et al., J. *Amer. Chem.* Soc. 87 (1965) 1995-2003].

Die Amidinverbindungen der Formel III können z. B. aus den entsprechenden Aminoverbindungen der Formel II gemäss bekannten Amidinsynthesen hergestellt werden, z. B. durch Umsetzung zunächst mit Orthoameisensäure-triethylester, vorzugsweise bei erhöhter Temperatur, und dann mit Ammoniak, vorzugsweise bei Raumtemperatur.

Verfahren (c): Geeignete Abgangsgruppen sind z. B. Mesylate oder Tosylate von Hydroxyverbindungen und Halogen. Die Herstellung von geigneten Pyrrolo[2,3-d]pyrimidinen der Formel IV ist literaturbekannt bzw. analog in in der Literatur beschriebenen Verfahren durchführbar. Die Umsetzung von Verbindungen der Formel IV mit Verbindungen der Formel V erfolgt in an sich bekannter Weise. So wird beispielsweise ein Mesylat der Formel V mit einem Pyrrolo[2,3-d]pyrimidin der Formel IV in Gegenwart einer Base, z. B. Kaliumcarbonat umgesetzt. Die Umsetzung erfolgt vorzugsweise bei erhöhter Temperatur, z. B. zwischen 50 °C und der Rückflusstemperatur des Reaktionsgemisches, insbesondere bei 60-80 °C, und vorteilhaft in einem inerten Lösungsmittel oder Lösungsmittelgemisch. Die Reaktion kann in vorteilhafter Weise durch Zusatz eines geeigneten Kronenethers beschleunigt werden. Bei einem weiteren Verfahren findet die Umsetzung in an sich bekannter Weise unter den Bedingungen der Phasentransfer-Katalyse (E. V. Dehmlow und S. S. Dehmlow, *Phase Transfer Catalysis,* 3rd ed., VCH, Weinheim, 1993) statt. Die Reaktanden der Formeln IV und V werden in einem geeigneten inerten Lösungsmittel oder Lösungsmittelgemisch gelöst, die zweite Phase wird durch eine konzentrierte wässerige Alkalihydroxidlösung, beispielsweise 30 proz. Natronlauge, gebildet. Vorteilhafterweise wird ein Phasentransfer-Katalysator, beispielsweise ein quaternäres Ammoniumhalogenid wie z. B. Tetrabutylammoniumbromid, zugesetzt.

Verfahren (d): Die Umsetzung einer Verbindung der Formel VI mit einem Amin erfolgt in an sich bekannter Weise. Sie wird vorteilhafterweise in einem inerten Lösungsmittel oder Lösungsmittelgemisch unter Kühlung, bei Umgebungstemperatur oder bei erhöhter Temperatur bis zur Rückflusstemperatur des Reaktionsgemisches, insbesondere unter Eiskühlung, bei Raumtemperatur oder erhöhter Temperatur bis zur Rückflusstemperatur. Gegebenenfalls wird die Umsetzung auch mit oder ohne Lösungsmittel im Bombenrohr durchgeführt. Verbindungen der Formel VI erhält man z. B. durch Umsetzung einer gegebenfalls geschützen Verbindung der Formel IV mit einer Verbindung der Formel Vla 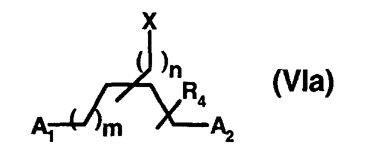 worin X eine geeignete Abgangsgruppe, beispielsweise ein Mesylat oder Tosylat, bedeutet und A₁ und A₂ für Reste stehen, die unter den Reaktionsbedingungen stabil sind, und anschliessend leicht in Abgangsgruppen Y₁ und Y₂ umgewandelt werden können. Beispielsweise können A₁ und A₂ Silyletherreste, insbesondere *tert*.-Butyldimethylsilyletherreste, sein, die sich nach der Umsetzung einer gegebenenfalls geschützten Verbindung IV mit einer Verbindung Vla in an sich bekannter Weise spalten lassen, worauf man eine gegebenenfalls geschützte Verbindung der allgemeinen Formel VI erhält, in der Y₁ und Y₂ für Hydroxylreste stehen. Diese Hydroxylreste lassen sich dann in sich bekannter Weise in andere Abgangsgruppen, z. B. Halogen, überführen. Die Umsetzung einer gegebenfalls geschützten Verbindung der Formel IV mit einer Verbindung der Formel VIa kann beispielsweise analog der für Vefahren c) beschriebenen Umsetzung einer Verbindung der Formel IV mit einer Verbindung der Formel V durchgeführt werden.

Verbindungen der Formel I können in andere Verbindungen der Formel I überführt werden.

So können z. B. in an sich bekannter Weise Substituenten im Arylrest R₁ ineinander umgewandelt werden.

Halogen-niederalkyl, z. B. Chlormethyl, etwa kann z. B. mit gegebenenfalls substituierten Niederalkanolen, Niederalkanthiolen oder Niederalkylaminen gemäss einer nukleophilen Substitutionsreaktion umgesetzt werden, wobei gegebenenfalls substituiertes Niederalkoxyniederalkyl, Niederalkylthio-niederalkyl bzw. Niederalkylamino-niederalkyl erhalten wird.

Hydroxy kann z. B. mit gegebenenfalls substituierten Halogenniederalkanen umgesetzt werden, wobei gegebenenfalls substituiertes Niederalkoxy erhalten wird. Hydroxy kann z. B. auch zunächst mit einem Dihalogen-niederalkan, z. B. 1-Brom-2-chlorethan, umgesetzt werden, wobei man Ω-Halogen-niederalkoxy erhält; dieses kann analog wie oben beschrieben mit gegebenenfalls substituierten Niederalkanolen, Niederalkanthiolen oder Niederalkylaminen gemäss einer nukleophilen Substitutionsreaktion umgesetzt werden, wobei gegebenenfalls substituiertes Niederalkoxy-niederalkoxy, Niederalkylthio-niederalkoxy bzw. Niederalkylamino-niederalkoxy erhalten wird.

Analog wie Hydroxy kann auch Mercapto wie im vorigen Absatz beschrieben alkyliert werden.

Niederalkylthiogruppen können durch gezielte Oxidation sowohl in Niederalkylsulfinyl- als auch Niederalkylsulfonylgruppen übertührt werden.

Aminogruppen und Hydroxygruppen können in bekannter Weise acyliert werden, wobei z. B. Niederalkanoylamino- bzw. Niederalkanoyloxy-Gruppen erhalten werden.

Carbonsäurereste können gemäss bekannten Derivatisierungsmethoden, wie z. B. der Veresterung oder Amidbildung, in Carbonsäure-Derivate, wie z. B. Niederalkoxycarbonyl, Aminocarbonyl, N-Niederalkylaminocarbonyl, N,N-Diniederalkylaminocarbonyl, Cyano oder Amidino, überführt werden. Umgekehrt können auch Carbonsäure-Derivate in freie Carbonsäuren umgewandelt werden, z. B. durch Verseifung.

Verbindungen der Formel I, worin R₂ Wasserstoff bedeutet, können durch Umsetzung mit einem Halogenierungsmittel, z. B. einem N-Halogen-succinimid, in Verbindungen der Formel I, worin R₂ Halogen ist, umgewandelt werden.

Substituenten in den Resten R₄ und R₅ können in an sich bekannter Weise in andere Substituenten umgewandelt werden.

So können Hydroxygruppen mit organischen oder anorganischen Säuren verestert oder mit Akoholen oder organischen Haliden verethert werden oder sie können reduktiv entfernt werden.

Wenn irgendwelche Zwischenprodukte störende reaktionsfähige Gruppen, z. B. Carboxy-, Hydroxy-, Mercapto- oder Aminogruppen, enthalten, können diese vorübergehend durch leichtabspaltbare Schutzgruppen geschützt werden. Die Auswahl von geeigneten Schutzgruppen, ihre Einführung und Entfernung sind an sich bekannt und sind z. B. in J.F.W. McOmie, *Protective Groups in Organic Chemistry,* Plenum Press, London, New York 1973 beschrieben, femer z. B. auch in T.W. Greene und P.G.M. Wuts, *Protective Groups in Organic Synthesis,* Wiley, New York et al. 1991, oder auch in P.J. Kocienski, *Protecting Groups*, Thieme, Stuutgart, New York 1994.

Salze von Verbindungen I können in an sich bekannter Weise hergestellt werden. So erhält man beispielsweise Säureadditionssalze von Verbindungen I durch Behandeln mit einer geeigneten Säure oder einem geeigneten lonenaustauscherreagens und Salze mit Basen durch Behandeln mit einer geeigneten Base oder einem geeigneten lonenaustauscherreagens. Salze von Verbindungen der Formel I können in üblicher Weise in die freien Verbindungen I überführt werden, Säureadditionssalze z. B. durch Behandeln mit einem geeigneten basischen Mittel oder einem geeigneten lonenaustauscherreagens und Salze mit Basen z. B. durch Behandeln mit einer geeigneten Säure oder einem geeigneten lonenaustauscherreagens.

Salze von Verbindungen I können in an sich bekannter Weise in andere Salze von Verbindungen I umgewandelt werden, Säureadditionssalze beispielsweise in andere Säureadditionssalze, z. B. durch Behandeln eines Salzes einer anorganischen Säure, wie eines Hydrochlorids, mit einem geeigneten Metallsalz, wie einem Natrium-, Barium- oder Silbersalz, einer Säure, z. B. mit Silberacetat, in einem geeigneten Lösungsmittel, in welchem ein sich bildendes anorganisches Salz, z. B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Infolge der engen Beziehung zwischen der Verbindung I in freier Form und in Form ihrer Salze sind im vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren oder als Gemisch derselben vorliegen. Dabei sind als reine Isomere z. B. reine Diastereoisomere erhältlich. Entsprechend können als Isomerengemische z. B. Diastereoisomerengemische vorliegen. Verfahrensgemäss oder anderweitig erhältliche Isomerengemische von Verbindungen I in freier Form oder in Salzform können in üblicher Weise in die Komponenten aufgetrennt werden, z. B. aufgrund der physikalisch-chemischen Unterschiede der Bestandteile in bekannter Weise durch fraktionierte Kristallisation, Destillation und/oder Chromatographie. Vorteilhaft isoliert man das wirksamere Isomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen. Neue Ausgangsstoffe und Zwischenprodukte, jeweils in freier Form oder in Salzform, für die Herstellung der Verbindungen I bzw. ihrer Salze, ihre Verwendung und Verfahren zu ihrer Herstellung bilden ebenfalls einen Gegenstand der Erfindung, wobei die Variable R die für die Verbindungen I angegebene Bedeutung hat.

Die Erfindung betrifft ebenfalls die Verwendung der Verbindungen I und ihrer pharmazeutisch verwendbaren Salze zur Behandlung von allergischen Zuständen und Erkrankungen, vorzugsweise in Form von pharmazeutisch verwendbaren Zubereitungen, insbesondere in einem Verfahren zur therapeutischen Behandlung des tierischen oder menschlichen Körpers, sowie ein solches Behandlungsverfahren.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich z. B. um solche zur enteralen, wie insbesondere oralen, ferner rektalen, Verabreichung, um solche zur parenteralen Verabreichung und um solche zur lokalen Verabreichung an Warmblüter, vor allem an Menschen, wobei der pharmakologische Wirkstoff allein oder zusammen mit üblichen pharmazeutischen Hilfsstoffen enthalten ist. Die pharmazeutischen Präparate enthalten (in Gewichtsprozenten) z. B. von etwa 0,001 % bis 100 %, vorzugsweise von etwa 0,1 % bis etwa 50 %, des Wirkstoffs.

Pharmazeutische Präparate zur enteralen bzw. parenteralen Verabreichung sind z. B. solche in Dosiseinheitsformen, wie Dragées, Tabletten, Kapseln oder Suppositorien, ferner Ampullen. Diese werden in an sich bekannter Weise, z. B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragee-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, femer Bindemittel, wie Stärkekleister, unter Verwendung z. B. von Mais-, Weizen-, Reis- oder Kartoffelstärke, Gelatine, Tragacanth, Methylcellulose und/oder Polyvinylpyrrolidon, und, wenn erwünscht, Sprengmittel, wie die obengenannten Stärken, femer Carboxymethylstärke, quervernetztes Polyvinylpyrrolidon, Agar oderAlginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten, Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragee-Ueberzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z. B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z. B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z. B. Sesamöl, oder synthetische Fettsäureester, z. B. Ethyloleat oder Triglyceride, verwendet, oder wässrige Injektionssuspensionen, welche viskositätserhöhende Stoffe, z. B. Natriumcarboxymethylcellulose, Sorbit und/oder Dextran, und gegebenenfalls auch Stabilisatoren enthalten.

Pharmazeutische Präparate zur lokalen Verabreichung sind z. B. für die topische Behandlung der Haut Lotionen, Crèmes und Salben, d. h. flüssige oder semifeste Oel-in-Wasseroder Wasser-in-Oel-Emulsionen, Fettsalben, die wasserfrei sind, Pasten, d. h. Crèmes und Salben mit sekretabsorbierenden Puderbestandteilen, Gele, die wässrig, wasserarm oder wasserfrei sind und aus quellbaren, gelbildenden Materialien bestehen, Schäume, d. h. in Aerosolform vorliegende flüssige Oel-in-Wasser-Emulsionen, welche aus Druckbehältern verabreicht werden, und eine wässerig-ethanolische Grundlage aufweisende Tinkturen, welche jeweils weitere übliche pharmazeutische Hilfsstoffe, wie Konservierungsmittel, enthalten können. Die Herstellung der pharmazeutischen Präparate zur lokalen Verabreichung erfolgt in an sich bekannter Weise durch Vermischung des Wirkstoffs mit den pharmazeutischen Hilfsstoffen, z. B. durch Lösen oder Suspendieren des Wirkstoffs in der Grundlage oder in einem Teil davon, falls notwendig. Zur Herstellung von Emulsionen, in denen der Wirkstoff in einer der flüssigen Phasen gelöst ist, wird der Wirkstoff in der Regel vor der Emulgierung in dieser gelöst; zur Herstellung von Suspensionen, in denen der Wirkstoff in der Emulsion suspendiert ist, wird der Wirkstoff nach der Emulgierung mit einem Teil der Grundlage vermischt und dann dem Rest der Formulierung beigegeben.

Die Dosierung des Wirkstoffs kann von verschiedenen Faktoren, wie Wirksamkeit und Wirkungsdauer des Wirkstoffs, Stärke der zu behandelnden Krankheit bzw. ihrer Symptome, Applikationsweise, Warmblüter-Spezies, -Geschlecht, -Alter, -Gewicht und/oder individuellem Zustand des Warmblüters, abhängen. Im Normalfall ist für einen etwa 75 kg schweren Warmblüter eine, z. B. orale, Tagesdosis von etwa 1 mg bis etwa 1000 mg, insbesondere von etwa 5 bis etwa 200 mg zu veranschlagen. Diese kann z. B. als Einmaldosis oder in mehreren Teildosen, z. B. solchen von 10 bis 100 mg, verabreicht werden.

Die vorstehend beschriebene Erfindung soll durch die nachfolgenden Beispiele illustriert werden, jedoch ohne sie auf diese einzuschränken. (Vor- und nachstehend haben folgende Abkürzungen - soweit nicht anders angegeben - die Bedeutungen: Smp., Schmelzpunkt, Std., Stunden; CDCl₃, Deuterochloroform; DMSO-d₆, Hexadeuterodimethylsulfoxid; CD₃OD, Deuteromethanol)

### Beispiel 1: 5-Phenyl-7H-pyrrolo[2,3-d]pyrimidin-4-yl-amin

In 150 ml Ethanol werden 4,3 g N-[3-Cyano-4-(4-methoxy-phenyl)-*1H*-pyrrol-2-yl]-formamidin suspendiert und mit 0,3 g Natriumethylat versetzt. Es wird 1 Std. unter Rückfluss gerührt, das Reaktionsgemisch auf Raumtemperatur abgekühlt und das Produkt abfiltriert. Smp.: 260-261 °C.
a) N-(2-Oxo-2-phenyl-ethyl)-acetamid: In 150 ml Tetrahydrofuran werden 25,0 g Phenacylaminhydrochlorid, 40,5 ml Triethylamin und 27,6 ml Acetanhydrid suspendiert. Es wird 2,5 Std. bei Raumtemperatur gerührt, anschliessend wird filtriert und das Tetrahydrofuran am Rotationsverdampfer abgezogen. Der Rückstand wird aus Diethylether kristallisiert. Smp.: 95-96 °C.
b) 2-Amino-4-phenyl-*1H*-pyrrol-3-carbonitril: In 100 ml Ethanol werden 0,9 g Natrium gelöst und 2,6 g Malonsäuredinitril zugegeben. Es wird 30 min bei 55 °C gerührt, dann werden 7,0 g N-(2-Oxo-2-Phenyl-ethyl)-acetamid zugegeben und 2 Std. bei 55 °C gerührt. Das Reaktionsgemisch wird auf Eis gegossen und das Produkt filtriert. ¹H-NMR (DMSO-d₆, ppm): 10,4 (s, 1H), 7,6-7,1 (m, 5H), 6,62 (s, 1H), 5,75 (s, 2H).
c) N-[3-Cyano-4-(4-methoxy-phenyl)-*1H*-pyrrol-2-yl]-formamidin: In 80 ml Orthoameisensäuretriethylester werden 6,0 g 2-Amino-4-Phenyl-*1H*-pyrrol-3-carbonitril gelöst und 1 Std. bei 140 °C gerührt. Der Orthoameisensäuretriethylester wird am Hochvakuum abgezogen und der Rückstand in mit Ammoniak gesättigtem Methanol gelöst. Es wird 24 Std. bei Raumtemperatur gerührt und danach filtriert. Das Produkt wird aus Ethanol umkristallisiert. Smp.: 238-239 °C.

### Beispiel 2: 5-(3-Methoxy-phenyl)-7H-pyrrolo[2,3-d]pyrimidin-4-yl-amin

In 100 ml Ethanol werden 0,43 g Natriumethylat gelöst und 5,0 g N-[3-Cyano-4-(3-methoxyphenyl)-*1H*-pyrrol-2-yl]formamidin zugegeben. Es wird 1 Std. unter Rückfluss gerührt, beim Abkühlen auf Raumtemperatur fällt das Produkt aus und wird filtriert. Smp.: 249-250 °C
a) 2-Bromo-1-(3-methoxy-phenyl)-ethanon: In 10 ml Diethylether werden 10 ml 3-Methoxyacetophenon gelöst und auf 5 °C abgekühlt, es werden 0,2 g Aluminiumtrichlorid zugegeben und die Mischung 5 min bei 5 °C gerührt. Danach werden 3,9 ml Brom bei 0-5 °C zugetropft und anschliessend 1 Std. bei 0-5 °C weitergerührt. Das Reaktionsgemisch wird in Ethylacetat gegossen, mit Wasser, gesättiger Ammoniumchloridlösung und gesättigter Kochsalzlösung gewaschen. Die organische Phase wird über wasserfreiem Natriumsulfat getrocknet und das Ethylacetat am Rotationsverdampfer abgezogen. Das Produkt wird aus Ether/Petrolether kristallisiert. Smp. 64-65°C.
b) 2-Azido-1-(3-Methoxy-phenyl)-ethanon: In 300 ml Toluol werden 12,0 g 2-Bromo-1-(4-methoxy-phenyl)-ethanon und 1,0 g Tricaprylmethylammoniumchlorid (Aliquot 366) vorgelegt und eine Lösung aus 13,6 g Natriumazid in 40 ml Wasser wird zugetropft. Es wird 1,5 Std. bei 50-55 °C gerührt. Das Reaktionsgemisch wird auf Raumtemperatur abgekühlt, die Wasserphase wird abgetrennt und mit Toluol extrahiert. Die organischen Phasen werden vereinigt und mit Wasser gewaschen, getrocknet und das Lösungsmittel wird am Rotationsverdampfer abgezogen. Flash-Chromatographie mit Diethylether/Petrolether als Fliessmittel ergibt das Produkt als Oel, ¹H-NMR (CDCl₃, ppm):7,5-7,1 (m, 4H), 4,55 (s, 2H), 3,9 (s, 3H).
c) 2-Amino-1-(3-methoxy-phenyl)ethanonhvdrochlorid: 7,7 g 2-Azido-1-(4-Methoxy-phenyl)-ethanon und 12 ml 4N Salzsäure werden in 150 ml Methanol gelöst und über 1,5 g Palladium/Kohle (10%) 1 Std. bei Normaldruck hydriert. Die Hydrierlösung wird filtriert und das Filtrat eingedampft. ¹H-NMR (DMSO-d₆, ppm): 8,5 (s(breit), 2H), 7,6-7,3 (m, 4H), 4,5 (q, 2H), 3,8 (s, 3H).
d) N-2-[2-(3-Methoxy-phenyl)-2-oxo-ethyl]acetamid: In 60 ml Tetrahydrofuran werden 7,60 g 2-Amino-1-(3-methoxy-phenyl)ethanonhydrochlorid, 10,5 ml Triethylamin und 7,1 ml Essigsäureanhydrid suspendiert und 2 Std. bei Raumtemperatur gerührt. Die Suspension wird filtriert und das Filtrat wird eingedampft. Der Rückstand wird in Ethylacetat aufgenommen und mit Wasser gewaschen. Die organische Phase wird über Natriumsulfat getrocknet und das Lösungsmittel am Rotationsverdampfer abgezogen. Dies ergibt einen kristallinen Rückstand, der mit Ether verrührt wird. Smp.: 109-110°C.
e) 2-Amino-4-(3-methoxy-phenyl)-*1H*-pyrrol-3-carbonitril: In 100 ml Ethanol werden 0,71g Natrium gelöst und 2,03 g Malonsäuredinitril zugegeben. Es wird 30 min bei 55 °C gerührt, dann werden 6,38 g N-2-[2-(3-Methoxy-phenyl)-2-oxo-ethyl]acetamid zugegeben und weitere 2 Std. bei 55 °C gerührt. Das Reaktionsgemisch wird auf Eis gegossen und das Produkt filtriert. Smp.:117-119°C.
f) N-[3-Cyano-4-(3-methoxy-phenyl)-*1H*-pyrrol-2-yl]formamidin: In 50 ml Orthoameisensäuretriethylester werden 5,30 g 2-Amino-4-(3-methoxy-phenyl)-*1H*-pyrrol-3-carbonitril gelöst und 1 Std. bei 140 °C gerührt. Der Orthoameisensäuretriethylester wird am Hochvakuum abgezogen und der Rückstand in mit Ammoniak gesättigtem Methanol gelöst. Es wird 20 Std. bei Raumtemperatur gerührt und danach filtriert. Das Produkt wird aus Ethanol umkristallisiert. Smp.: 188-190 °C.

### Beispiel 3: 5-(4-Benzyloxy-phenyl)-7H-pyrrolo[2,3-d]pyrimidin-4-yl-amin

In 200 ml Orthoameisensäuretriethylester werden 15,2 g 2-Amino-4-(4-benzyloxy-phenyl)-*1H*-pyrrol-3-carbonitril gelöst und 1 Std. bei 140 °C gerührt. Der Orthoameisensäuretriethylester wird am Hochvakuum eingedampft und der Rückstand in 500 ml mit Ammoniak gesättigtem Methanol gelöst. Es wird 20 Std. bei Raumtemperatur gerührt. Die Suspension wird filtriert und die violetten Kristalle intensiv mit Methanol gewaschen. Smp.: 220 °C (Zersetzung).
a) 1-[4-(Benzyloxy)phenyl]ethanon: Zu einer Suspension von 50,0 g 4-Hydroxyacetophenon und 76,0 g Kaliumcarbonat in 600 ml Aceton werden 48 ml Benzylbromid zugetropft. Es wird 20 Std. unter Rückfluss gerührt. Das Reaktionsgemisch wird filtriert, das Filtrat eingedampft und der Rückstand mit Petrolether digeriert. ¹H-NMR (CDCl₃, ppm): 7,55 (m, 2H), 7,46 (m, 6H), 7,17 (ddd, 1H), 5,11 (s, 2H), 2,57 (s, 3H).
b) 2-Bromo-1-(4-benzyloxy-phenyl)-ethanon: In 580 ml Ethylacetat werden 173 g Kupfer(II)-bromid suspendiert und auf Rückflusstemperatur erhitzt. Dann wird eine Lösung von 1-[4-(Benzyloxy)phenyl]ethanon in 330 ml Chloroform innert 40 min zugetropft. Es wird 2 Std. unter Rückfluss gerührt. Die Suspension wird auf Raumtemperatur gekühlt und filtriert. Das Filtrat wird eingedampft und das Produkt mit Flash-Chromatographie (Dichlormethan/Petrolether 1:1) gereinigt. Smp.: 58-59°C.
c) 2-Amino-1-(4-benzyloxy-phenyl)ethanonhvdrochlorid: In 1 Chloroform werden 50,0 g 2-Bromo-1-(4-benzyloxy-phenyl)-ethanon gelöst und mit 35 g Hexamethylentetramin versetzt.
   Es wird 20 Std. bei Raumtemperatur gerührt. Das Produkt wird abfiltriert, in 300 ml Ethanol/100 ml konz. Salzsäure gelöst und 2 Std. unter Rückfluss gerührt. Das Reaktionsgemisch wird auf Raumtemperatur abgekühlt und das Produkt abfiltriert. Smp.: 237-240 °C (Zersetzung).
d) N-2[2-(4-Benzyloxy-phenyl)-2-oxo-ethyl]acetamid: In 300 ml Tetrahydrofuran werden 28,2 g 2-Amino-1-(4-benzyloxy-phenyl)ethanonhydrochlorid suspendiert, 28,2 ml Triethylamin und 11,5 ml Essigsäureanhydrid werden zugegeben. Das Reaktionsgemisch wird 3 Std. bei Raumtemperatur gerührt, filtriert und die Mutterlauge am Rotationsverdampfer eingedampft. Der Rückstand wird aus Diethylether mit Petrolether ausgefällt, 1 H-NMR (CDCl₃, ppm): 7,8 (d, 2H), 7,4 (m, 5H), 7,02 (d, 2H), 6,6 (t,1H), 5,18 (s, 2H), 4,7 (d, 2H), 2,1 (s, 3H).
e) 2-Amino-4-(4-Benzyloxy-phenyl)-*1H*-pyrrol-3-carbonitril: In 300 ml Ethanol werden 1,5 g Natrium gelöst und 4,5 g Malonsäuredinitril zugegeben. Es wird 30 min bei 40 °C gerührt, dann werden 16,1 g N-2[2-(4-Benzyloxy-phenyl)-2-oxo-ethyl]acetamid zugegeben und weitere 18 Std. bei 40 °C gerührt. Das Reaktionsgemisch wird auf Raumtemperatur abgekühlt und das Produkt filtriert. ¹H-NMR (DMSO-d₆, ppm): 10,3 (s, 1H), 7,48 (d, 2H), 7,35 (m, 5H), 7,0 (d, 2H), 6,4 (s, 1 H), 5,7 (s, 2H), 5,1 (s, 2H).

In Analogie zu den obigen Beispielen werden ausgehend von jeweiligen geeignet substituierten Acetophenon-Derivaten die entsprechenden Pyrrolopyrimidine hergestellt:

### Beispiel 4: 5-(4-Methoxy-phenyl)-7H-pyrrolo[2,3-d]pyrimidin-4-yl-amin, Smp.: 278-281 °C.

### Beispiel 5: 5-(3-Benzyloxy-phenyl)-7H-pyrrolo[2,3-d]pyrimidin-4-yl-amin, Smp.: 241-243°C.

### Beispiel 6: 5-(3-Fluor-phenyl)-7H-pyrrolo[2,3-d]pyrimidin-4-yl-amin

### Beispiel 7: 5-(4-Fluor-phenyl)-7H-pyrrolo[2,3-d]pyrimidin-4-yl-amin

### Beispiel 8: 5-(3-Chlor-phenyl)-7H-pyrrolo[2,3-d]pyrimidin-4-yl-amin

### Beispiel 9: 5-(4-Chlor-phenyl)-7H-pyrrolo[2,3-d]pyrimidin-4-yl-amin

### Beispiel 10: 5-(3-Brom-phenyl)-7H-pyrrolo[2,3-d]pyrimidin-4-yl-amin

### Beispiel 11: 5-(4-Brom-phenyl)-7H-pyrrolo[2,3-d]pyrimidin-4-yl-amin

### Beispiel 12: 5-p-Toluyl-7H-pyrrolo[2,3-d]pyrimidin-4-yl-amin

### Beispiel 13: 5-m-Toluyl-7H-pyrrolo[2,3-d]pyrimidin-4-yl-amin

### Beispiel 14: 5-(4-Trifluormethyl-phenyl)-7H-pyrrolo[2,3-d]pyrimidin-4-yl-amin

### Beispiel 15: 5-(3-Trifluormethyl-phenyl)-7H-pyrrolo[2,3-d]pyrimidin-4-yl-amin

Die Herstellung von (*2R*/*4S*)-4-(Toluyl-4-sulfonyloxy)-pyrrolidin-1,2-dicarbonsäure-1-*tert*.butylester-2-ethylester und (*2R*/*4R*)-4-(Toluyl-4-sulfonyloxy)-pyrrolidin-1,2-dicarbonsäure-1-*tert*.-butylester-2-ethylester erfolgt nach G. L. Baker et al., J. *Org. Chem.* (1981), 46, 2954.

### Beispiel 16: (2R/4S)-4-(4-Amino-5-phenyl-pyrrolo[2,3-d]pyrimidin-7-yl)-pyrrolidin-1,2-dicarbonsäure-1-tert.-butylester-2-ethylester

In 5 ml Dimethylformamid werden 0,540 g 5-Phenyl-*7H*-pyrrolo[2,3-d]pyrimidin-4-yl-amin, 0,70 g Kaliumcarbonat und 0,670 g 18-Krone-6-ether suspendiert und 15 Minuten bei 70 °C gerührt. Danach wird 0,420 g (*2R*/*4R*)-4-(Toluyl-4-sulfonyloxy)-pyrrolidin-1,2-dicarbonsäure-1-*tert*.-butylester-2-ethylester zugegeben und die Mischung weitere 6 Stunden bei 70 °C gerührt. Das Reaktionsgemisch wird auf Raumtemperatur abgekühlt, filtriert und dann in Ethylacetat aufgenommen. Die Ethylacetatlösung wird dreimal mit Wasser gewaschen, über Natriumsulfat getrocknet und das Lösungsmittel am Rotationsverdampfer abgezogen. Der Rückstand wird mit Flash-Chromatographie (Ethylacetat/Petrolether 10:1) gereinigt. ¹H-NMR (CDCl₃, ppm): 8,30 (s, 1H), 7,50 -7,30 (m, 5H), 6,95 (s, 1H), 5,5 (m, 1H), 5,22 (s, 2H), 4,62 - 4,42 (m, 1H), 4,24 (q, 2H), 4,12 - 3,80 (m, 2H), 2,75 (m, 1H), 2,50 (m, 1H), 1,48 (s, 9H), 1,30 (t, 3H).

### Beispiel 17: (2R/4S)-4-(4-Amino-5-phenyl-pyrrolo[2,3-d]pyrimidin-7-yl)-pyrrolidin-2-carbonsäure-ethylester

0,500 g (*2R*/*4S*)-4-(4-Amino-5-phenyl-pyrrolo[2,3-d]pyrimidin-7-yl)-pyrrolidin-1,2-dicarbonsäure-1-*tert*.-butylester-2-ethylester (Beispiel 16) werden in 5 ml absolutem Tetrahydrofuran gelöst, mit 15 ml 4 M Chlorwasserstoff in Diethylether versetzt und 3 Stunden bei Raumtemperatur gerührt. Das Produkt wird filtriert und am Hochvakuum getrocknet. Man erhält das Dihydrochlorid der Titelverbindung. Smp.: 176 -178°C.

### Beispiel 18: (2R/4S)-4-(4-Amino-5-phenyl-pyrrolo[2,3-d]pyrimidin-7-yl)-pyrrolidin-2-hydroxymethyl-pyrrolidin-1-carbonsäure-tert.-butylester

In 15 ml Diethylether werden 0,200 g (*2R*/*4S*)-4-(4-Amino-5-phenyl-pyrrolo[2,3-d]pyrimidin-7-yl)-pyrrolidin-1,2-dicarbonsäure-1-*tert*.-butylester-2-ethylester (Beispiel 16) gelöst und auf 0 °C abgekühlt. Dann werden 0,03 ml Methanol und 0,015 g Lithiumborhydrid zugegeben und 2 Stunden unter Rückfluss gerührt. Das Reaktionsgemisch wird mit gesättigter Ammoniumchloridlösung versetzt und mit Ethylacetat extrahiert. Die organische Phase wird abgetrennt, über Natriumsulfat getrocknet und das Lösungsmittel am Rotationsverdampfer abgezogen. Der Rückstand wird mit Flash-Chromatographie (Dichlormethan/Methanol 10:0,4) gereinigt. ¹H-NMR (CDCl₃, ppm): 8,31 (s, 1H), 7,5 (m, 5H), 6,92 (s, 1H), 5,5 (m, 1H), 5,20 (s, 2H), 4,3 (m, 1H), 3,9 (m, 1H), 3,8 (m, 1H), 2,5 (m, 1H), 2,22 (m, 1H), 1,88 (s(breit), 1H), 1,48 (s, 9H).

### Beispiel 19: (2R/4S)-[4-(4-Amino-5-phenyl-pyrrolo[2,3-d]pyrimidin-7-yl)-pyrrolidin-2-yl]-methanol

In 1 ml Tetrahydrofuran werden 0,080 g (*2R*/*4S*)-4-(4-Amino-5-phenyl-pyrrolo[2,3-d]pyrimidin-7-yl)-pyrrolidin-2-hydroxymethyl-pyrrolidin-1-carbonsäure-*tert*.-butylester (Beispiel 18) gelöst, mit 8 ml 4 M Chlorwasserstoff in Diethylether versetzt und 2 Stunden bei Raumtemperatur gerührt. Das Produkt wird filtriert und am Hochvakuum getrocknet. Man erhält das Dihydrochlorid der Titelverbindung. ¹H-NMR (DMSO-d₆, ppm): 8,42 (s, 1H), 7,88 (s, 1H), 7,50 (m, 5H), 5,52 (m, 1H), 4,05 (m, 1H), 3,75 (m, 1H), 3,58 (m, 1H), 2,52 (m, 1H), 2,41 (m, 1H).

### Beispiel 20: (2R/4S)-4-[4-Amino-5-(4-hydroxy-phenyl)-pyrrolo[2,3-d]pyrimidin-7-yl]-pyrrolidin-1,2-dicarbonsäure-1-tert.-butylester-2-ethylester

0,050 g (*2R*/*4S*)-4-[4-Amino-5-(4-benzyloxy-phenyl)-pyrrolo[2,3-d]pyrimidin-7-yl]-pyrrolidin-1,2-dicarbonsäure-1-*tert*.-butylester-2-ethylester werden in 5 ml Methanol gelöst und über 0,010 g Palladium/Kohle (10%) 18 Stunden bei Normaldruck hydriert. Der Katalysator wird abfiltriert, das Filtrat eingedampft und der Rückstand mit Flash-Chromatographie (Dichlormethan/Methanol 10: 0,4) gereinigt. ¹H-NMR (CDCl₃, ppm): 8,32 (s, 1H), 7,32 -7,25 (m, 3H), 6,95 (d, 2H), 6,88 (d, 1H), 5,5 (m, 1H), 5,20 (s, 2H), 4,62 - 4,48 (m, 1H), 4,25 ( m, 3H), 4,19 (m, 1H), 2,75 (m, 1H), 2,52 (m, 1H), 1,48 (s, 9H), 1,25 (t, 3H).
a) (*2R*/*4S*)-4-[4-Amino-5-(4-benzyloxy-phenyl)-pyrrolo[2,3-d]pyrimidin-7-yl]-pyrrolidin-1,2-dicarbonsäure-1-*tert*.-butylester-2-ethylester: Wird ausgehend von 5-(4-Benzyloxy-phenyl)-*7H*-pyrrolo[2,3-d]pyrimidin-4-yl-amin (Beispiel 3) und (*2R*/*4R*)-4-(Toluyl-4-sulfonyloxy)-pyrrolidin-1,2-dicarbonsäure-1-*tert.*-butylester-2-ethylester analog Beispiel 16 hergestellt. ¹H-NMR (CDCl₃, ppm): 8,30 (s, 1H), 7,50 -7,35 (m, 7H), 7,08 (d, 2H), 6,89 (d, 1H), 5,50 (m, 1H), 5,18 (s, 2H), 5,11 (s, 2H), 5,60 - 5,42 (m, 1H), 4,25 (q, 2H), 4,12 (m, 1H), 3,96 -3,75 (m, 1H), 2,75 (m, 1H), 2,50 (m, 1H), 1,49 (s, 9H), 1,28 (t, 3H).

### Beispiel 21: (2R/4S)-4-[4-Amino-5-(4-hydroxy-phenyl)-pyrrolo[2,3-d]pyrimidin-7-yl-pyrrolidin-2-carbonsäure-ethylester

In 3 ml Tetrahydrofuran werden 0,183 g (*2R*/*4S*)-4-[4-Amino-5-(4-hydroxy-phenyl)-pyrrolo-[2,3-d]pyrimidin-7-yl]-pyrrolidin-1,2-dicarbonsäure-1-*tert*.-butylester-2-ethylester gelöst, 20 ml 4 M Chlorwasserstoff in Diethylether werden zugegeben und es wird 16 Stunden bei Raumtemperatur gerührt. Die resultierende Suspension wird filtriert und das Produkt mit Diethylether gewaschen. Man erhält das Dihydrochlorid der Titelverbindung. ¹H-NMR (DMSO-d₆, ppm): 9,8 (s(breit), 1H), 8,5 (s, 1H), 7,9 (s, 1H), 7,3 (d, 2H), 6,9 (d, 2H), 5,6 (m, 1H), 4,9 (dd, 1H), 4,29 (q, 2H), 3,9 (m, 1H), 3,60 (m, 1H), 2,70 (m, 2H), 1,28 (t, 3H).

### Beispiel 22: (2R/4S)-4-[4-Amino-5-(4-hydroxy-phenyl)-pyrrolo[2,3-d]pyrimidin-7-yl-2-hydroxymethyl-pyrrolidin-1-carbonsäure-tert.-butylester

Wird ausgehend von (*2R*/*4S*)-4-[4-Amino-5-(4-benzyloxy-phenyl)-pyrrolo[2,3-d]pyrimidin-7-yl-2-hydroxymethyl-pyrrolidin-1-carbonsäure-*tert*.-butylester analog Beispiel 20 hergestellt, ¹H-NMR (CDCl₃, ppm): 10,5 (s, 1H), 8,3 (s, 1H), 7,3 (d, 2H), 6,95 (d, 2H), 6,85 (s, 1H), 5,58 (m, 1H), 5,22 (s, 2H), 4,28 (m, 1H), 4,0 - 3,62 (m, 4H), 2,5 (m, 1H), 2,22 (m, 1H), 1,5 (s, 9H).
a) (*2R*/*4S*)-4-[4-Amino-5-(4-benzyloxy-phenyl)-pyrrolo[2,3-d]pyrimidin-7-yl-2-hydroxymethyl-pyrrolidin-1-carbonsäure-*tert*.-butylester: Wird ausgehend von (*2R*/*4S*)-4-[4-Amino-5-(4-benzyloxy-phenyl)-pyrrolo[2,3-d]pyrimidin-7-yl]pyrrolidin-1,2-dicarbonsäure-1-*tert*.-butylester-2-ethylester (Beispiel 20a) analog Beispiel 18 hergestellt. ¹H-NMR (CDCl₃, ppm): 8,32 (s, 1H), 7,5 - 7,3 (m, 7H), 7,1 (d, 2H), 6,9 (s, 1H), 5,5 (m, 1H), 5,12 (s, 2H), 4,25 (m, 1H), 3,98 - 3,70 (m, 4H), 2,5 (m, 1H), 2,2 (m, 1H), 1,5 (s, 9H).

### Beispiel 23: (2R/4S)-4-[4-Amino-7-(5-hydroxymethyl)-pyrrolidin-3-yl)-7H-pyrrolo[2,3-d]pyrimidin-5-yl]-phenol

0,034 g (*2R*/*4S*)-4-[4-Amino-5-(4-benzyloxy-phenyl)-pyrrolo[2,3-d]pyrimidin-7-yl-pyrrolidin-2-carbonsäure-ethylester werden in 5 ml Methanol gelöst und über 0,010 g Palladium/Kohle (10%) 17 Stunden bei Normaldruck hydriert. Der Katalysator wird abfiltriert, das Filtrat eingedampft und der Rückstand mit Diethylether verfestigt. ¹H-NMR (CD₃OD, ppm): 8,38 (s, 1H), 7,52 (s, 1H), 7,32 (d, 2H), 6,92 (d, 2H), 5,62 (m, 1H), 4,30 (m, 1H), 4,00 - 3,88 (m, 4H), 2,60 (m, 2H).
a) (*2R*/*4S*)-4-(4-Amino-5-(4-benzyloxy-phenyl)pyrrolo[2,3-d]pyrimidin-7-yl]-2-hydroxymethyl-pyrrolidin-1-carbonsäure-*tert*.-butylester: Wird ausgehend von (*2R*/*4S*)-4-[4-Amino-5-(4-benzyloxy-phenyl)-pyrrolo[2,3-d]pyrimidin-7-yl]-pyrrolidin-1,2-dicarbonsäure-1-*tert*.-butylester-2-ethylester (Beispiel 20 a) analog Beispiel 18 hergestellt. ¹H-NMR (CDCl₃, ppm): 8,52 (s, 1H), 7,52 - 7,32 (m, 7H), 7,1 (d, 2H), 6,95 (d, 1H),5,50 (m, 1H), 5,13 (s, 2H), 4,62 -4,42 (m, 2H), 4,28 (m, 2H), 4,10 (m, 1H), 3,95 - 3,70 (m, 1H), 2,75 (m, 1H), 2,50 (m, 1H), 1,49 (s, 9H).
b) (*2R*/*4S*)-{4-(4-Amino-5-(4-benzyloxy-phenyl)-pyrrolo[2,3-d]pyrimidin-7-yl]-pyrrolidin-2-yl}methanol: In 4 ml Tetrahydrofuran werden 0,100 g (*2R*/*4S*)-4-[4-Amino-5-(4-benzyloxyphenyl)-pyrrolo[2,3-d]pyrimidin-7-yl]-pyrrolidin-1,2-dicarbonsäure-1-*tert*.-butylester gelöst und mit 10 ml 4 M Chlorwasserstoff in Diethylether versetzt und 1 Stunde bei Raumtemperatur gerührt. Das Produkt wird filtriert und am Hochvakuum getrocknet. Man erhält das Dihydrochlorid der Titelverbindung. ¹H-NMR (CD₃OD, ppm): 8,4 (s, 1H); 7,60 (s, 1H), 7,5 - 7,10 (m, 9H), 5,65 (m, 1H), 5,18 (s, 2H), 4,32 (m, 1H), 4,00 - 3,65 (m, 4H), 2,60 (m, 2H).

### Beispiel 24: (2R/4S)-4-(4-Amino-5-phenyl-pyrrolo[2,3-d]pyrimidin-7-yl)-1-(2,2-dimethylpropionyl)-pyrrolidin-2-carbonsäureethylester

0,130 g (*2R*/*4S*)-4-(4-Benzyloxycarbonylamino-5-phenyl-pyrrolo[2,3-d]pyrimidin-7-yl)-1-(2,2-dimethyl-propionyl)-pyrrolidin-2-carbonsäureethylester werden in 8 ml Methanol gelöst und über 0,030 g Palladium/Kohle (10%) 1 Stunde bei Normaldruck hydriert. Der Katalysator wird abfiltriert, das Filtrat eingedampft und der Rückstand wird mit Flash-Chromatographie (Dichlormethan/Methanol 10:0,4) gereinigt. ¹H-NMR (DMSO-d₆, ppm): 8,2 (s, 1H), 7,55 - 7,30 (m, 6H), 6,15 (s(breit), 2H), 5,99 (m, 1H), 4,62 (m, 1H), 4,30 - 3,98 (m, 4H), 2,78 (m, 1 H), 2,30 (m, 1H), 1,22 (t, 3H), 1,10 (m, 9H).
a) (*2R*/*4S*)-4-(4-Benzyloxycarbonylamino-5-phenyl-pyrrolo[2,3-d]pyrimidin-7-yl)-pyrrolidin-1,2-dicarbonsäure-1-*tert.*-butylester-2-ethylester: In 30 ml Dichlormethan werden 1,51 g Carbobenzoxyimidazolid (Z-Imidazol) gelöst und auf 0 °C abgekühlt, dann wird 1,41 g Triethyloxoniumtetrafluoroborat zugegeben, 1 Std. bei 0 °C und 3 Stunden bei Raumtemperatur gerührt. Dann wird 0,560 g (*2R*/*4S*)-4-(4-Amino-5-phenyl-pyrrolo[2,3-d]pyrimidin-7-yl)-pyrrolidin-1,2-dicarbonsäure-1-*tert*.-butylester-2-ethylester (Beispiel 16) zugegeben und 20 Std. bei Raumtemperatur gerührt. Das Reaktionsgemisch wird in 150 ml Dichlormethan aufgenommen, zweimal mit gesättigter Natriumhydrogensulfatlösung, zweimal mit Wasser und einmal mit gesättigter Natriumchloridlösung extrahiert. Die organische Phase wird über Natriumsulfat getrocknet, filtriert und das Lösungsmittel am Rotationsverdampfer abgezogen. Der Rückstand wird mit Flash-Chromatographie (Diethylether/Petrolether 8:2) gereinigt. ¹H-NMR (CDCl₃, ppm): 8,70 (s, 1H), 7,50 - 7,32 (m, 10H), 7,23 (s, 1H), 5,55 (m, 1H), 5,08 (s, 2H), 4,48 (m, 1H), 4,25 - 4,10 (m, 3H), 3,80 (m, 1H), 2,97 (m, 1H), 2,40 (m, 1H), 1,45 (s, 9H), 1,22 (t, 3H).
   Z-lmidazol *[CAS-Reg-Nr.: 22129-07-3]* wird aus Z-Chlorid und Imidazol nach B.E. Watkins, H. Rapoport, *J. Org. Chem.* (1982), **47**, 4471 - 4477 hergestellt.
b) (*2R*/*4S*)-4-(4-Benzyloxycarbonyl-amino-5-phenyl-pyrrolo[2,3-d]pyrimidin-7-yl)-pyrrolidin-2-carbonsäureethylester: Wird aus (*2R*/*4S*)-4-(4-Benzyloxycarbonylamino-5-phenyl-pyrrolo-[2,3-d]pyrimidin-7-yl)-pyrrolidin-1,2-dicarbonsäure-1-*tert*.-butylester-2-ethylester analog Beispiel 19 hergestellt. Man erhält das Hydrochlorid der Titelverbindung. ¹H-NMR (DMSO-d₆, ppm): 8,28 (s, 1H), 8,00 (s, 1H), 7,60 - 7,30 (m, 10H), 5,80 (m, 1H), 5,30 (s, 2H), 4,70 (m, 1H), 4,32 (m, 2H), 4,10 -3,90 (m, 2H), 3,20 (m, 1H), 2,80 (m, 1H), 1,32 (t, 3H).
c) (*2R*/*4S*)-4-(4-Benzyloxycarbonylamino-5-phenyl-pyrrolo[2,3-d]pyrimidin-7-yl)-1-(2,2-dimethyl-propionyl)-pyrrolidin-2-carbonsäureethylester: In 4 ml Tetrahydrofuran/Dimethylformamid (1:1) werden 0,250 g (*2R*/*4S*)-4-(4-Benzyloxycarbonyl-amino-5-phenyl-pyrrolo[2,3-d]-pyrimidin-7-yl)-pyrrolidin-2-carbonsäureethylesterhydrochlorid suspendiert, danach werden 0,140 ml Triethylamin und 0,06 ml Pivaloylchlorid zugegeben. Es wird 3 Stunden bei Raumtemperatur gerührt. Das Reaktionsgemisch wird in Ethylacetat aufgenommen und dreimal mit Wasser gewaschen. Die organische Phase wird über wasserfreiem Natriumsulfat getrocknet, das Lösungsmittel wird am Rotationsverdampfer abgezogen und der Rückstand mit Flash-Chromatographie (Dichlormethan/Methanol 10:0,2) gereinigt. ¹H-NMR (CDCl₃, ppm): 8,75 (s, 1H), 7,52 - 7,31 (m, 10H), 7,09 (s, 1H), 5,65 (m, 1H), 5,10 (s, 2H), 4,80 (m, 1H), 4,40 - 4,20 (m, 2H), 4,12 (q, 2H), 2,75 (m, 1H), 2,45 (m, 1H), 1,30 - 1,18 (m, 12H).

Analog Beispiel 24 werden die Beispiele 25 - 29 hergestellt:

### Beispiel 25: (2R/4S)-4-(4-Amino-5-phenyl-pyrrolo[2,3-d]pyrimidin-7-yl)-1-(3.3-dimethylbutyryl)-pyrrolidin-2-carbonsäure-ethylester

¹H-NMR (CDCl₃, ppm): 8,28 (s, 1H), 7,5 -7,35 (m, 5H), 6,9 (s, 1H), 5,70 (s(breit), 2H), 5,55 (m, 1H), 4,80 (m, 1H), 4,3 - 4,2 (m, 3H), 3,90 (m, 1H), 2,80 (m, 1H), 2,50 (m, 1H), 2,2 (q, 2H), 1,30 (t, 3H), 1,01 (s, 9H).

### Beispiel 26: (2R/4S)-4-[4-Amino-5-(4-hydroxyphenyl)-pyrrolo[2,3-d]pyrimidin-7-yl]-1-(3,3-dimethyl-butyryl)-pyrrolidin-2-carbonsäure-ethylester

¹H-NMR (CDCl₃, ppm): 8,30 (s, 1H), 7,30 (d, 2H), 6,98 (d, 2H), 6,80 (s, 1H), 5,60 (m, 1H), 5,40 (s(breit), 2H), 4,80 (m, 1H), 4,30 (m, 3H), 3,95 (m, 1H), 2,80 (m, 1H), 2,55 (m, 1H), 2,22 (q, 2H), 1,30 (t, 3H), 1,02 (s, 9H).

### Beispiel 27: (2R/4S)-4-(4-Amino-5-phenyl-pyrrolo[2,3-d]pyrimidin-7-yl)-1-tert.-butoxyacetylpyrrolidin-2-carbonsäure-ethylester

¹H-NMR (CDCl₃, ppm): 8,3 (s, 1H), 7,5 - 7,35 (m, 5H), 7,0 (s, 1H), 5,55 (m, 1H), 5,10 (s, 2H), 4,75 (m, 1H), 4,32 - 4,00 (m, 6H), 2,78 (m, 1H), 2,5 (m, 1H), 1,28 (t, 3H), 1,10 (s, 9H).

### Beispiel 28: (2R/4S)-4-(4-Amino-5-phenyl-pyrrolo[2,3-d]pyrimidin-7-yl)-1-(2-tert.-butoxycarbonylamino-3-methyl-pentanoyl)-pyrrolidin-2-carbonsäure-ethylester

¹H-NMR (CDCl₃, ppm): 8,30 (s, 1H), 7,50 -7,32 (m, 5H), 6,9 (s, 1H), 5,55 (m, 1H), 5,27 (m, 3H), 4,75 (m, 1H), 4,42 - 4,03 (m, 4H), 2,95 (m, 1H), 2,50 (m, 1H), 1,48 (s, 9H), 1,39 - 1,28 (m, 5H), 1,10 (m, 1H), 0,9 (d, 3H), 0,8 (t, 3H).

### Beispiel 29: (2R/4S)-1-(2-Amino-3-methyl-pentanoyl)-4-(4-amino-5-phenyl-pyrrolo[2,3-d]-pyrimidin-7-yl)-pyrrolidin-2-carbonsäure-ethylester

¹H-NMR (DMSO-d₆, ppm): 8,50 (s, 1H), 8,20 (s(breit), 2H), 7,80 (s, 1H), 7,50 - 7,35 (m, 5H), 5,55 (m, 1H), 4,72 (m, 1H), 4,38 -3,90 (m, 4H), 3,00 (m, 1H), 2,50 (m, 1H), 1,25 (t, 3H), 1,0 (m, 1H), 0,90 (d, 3H), 0,70 (t, 3H).

### Beispiel 30: (2R/4S)-1-[4-(4-Amino-5-phenyl-pyrrolo[2,3-d]pyrimidin-7-yl)-2-hydroxymethylpyrrolidin-1-yl]-2,2-dimethyl-propan-1-on

Wird aus (*2R*/*4S*)-{7-[1-(2,2-Dimethyl-propionyl)-5-hydroxymethyl-pyrrolidin-3-yl]-5-phenyl-7H-pyrrolo[2,3-d]pyrimidin-4-yl}-carbaminsäure-benzylester analog Beispiel 24 hergestellt. ¹H-NMR (CDCl₃, ppm): 8,3 (s, 1H), 7,52 - 7,38 (m, 5H), 6,9 (s, 1H), 2H), 5,55 (m, 1H), 5,20 (s(breit), 4,75 (m, 1H), 4,18 - 3,70 (m, 4H), 2,60 (m, 1H), 2,25 (m, 1H), 1,70 (s (breit),1H), 1,00 (s, 9H).
a) (*2R*/*4S*)-{7-[1-(2,2-Dimethyl-propionyl)-5-hydroxymethyl-pyrrolidin-3-yl]-5-phenyl-*7H*pyrrolo[2,3-d]pyrimidin-4-yl}-carbaminsäure-benzylester: Wird analog Beispiel 18 aus (*2R*/*4S*)-4-(4-Benzyloxycarbonylamino-5-phenyl-pyrrolo[2,3-d]pyrimidin-7-yl)-1-(2,2-dimethylpropionyl)-pyrrolidin-1,2-dicarbonsäure-1-ethylester (Beispiel 24c) hergestellt. ¹H-NMR (CDCl₃, ppm): 8,72 (s, 1H), 7,50 - 7,30 (m, 10H), 7,02 (s, 1H), 5,59 (m, 1H), 5,08 (s, 2H), 4,75 (m, 1H), 4,20 - 3,70 (m, 4H), 2,51 (m, 1H), 2,25 (m, 1H), 1,75 (s(breit), 1H), 1,12 (s, 9H).

Analog Beispiel 30 werden die Beispiele 31 - 34 hergestellt:

### Beispiel 31: (2R/4S)-1-[4-(4-Amino-5-phenyl-pyrrolo[2,3-d]pyrimidin-7-yl)-2-hydroxymethylpyrrolidin-1-yl]-3,3-dimethyl-butan-1-on

¹H-NMR (CDCl₃, ppm): 8,30 (s, 1H), 7,5 - 7,34 (m 5H), 6,97 (s, 1H), 5,52 (m, 1H), 5,28 (s, 2H), 4,71 (m, 1H), 4,35 (m, 1H), 4,10 (m, 1H), 3,95 - 3,70 (m, 2H), 2,60 (m, 1H), 2,30 (m, 1H), 2,20 (q, 2H), 1,02 (s, 9H).

### Beispiel 32: (2R/RS)-1-{4-Amino-5-(4-hydroxy-phenyl)-pyrrolo[2,3-d]pyrimidin-7-yl]-2-hydroxymethyl-pyrrolidin-1-yl}-3,3dimethyl-butan-1-on

¹H-NMR (CD₃OD, ppm): 8,15 (s, 1H), 7,30 (d, 2H), 7,15 (s, 1H), 6,88 (d, 2H), 5,51 (m, 1H), 4,50 (m, 1H), 4,10 - 3,60 (m, 4H), 2,70 - 2,50 (m, 2H), 2,25 (q, 2H), 0,99 (s, 9H).

### Beispiel 33: (2R/4S)-1-[4-(4-Amino-5-phenyl-pyrrolo[2,3-d]pyrimidin-7-yl)-2-hydroxymethylpyrrolidin-1-yl]-2-tert.-butoxy-ethanon

¹H-NMR (CDCl₃, ppm): 8,70 (s, 1H), 7,50 - 7,40 (m, 5H), 7,10 (s, 1H), 5,58 (m, 1H), 4,52 (m, 1H), 4,32 (m, 1 H), 4,10 (m, 1 H), 4,00 (m, 2H), 3,90 (m, 1 H), 3,72 (m, 1 H), 2,52 (m, 1 H), 2,30 (m, 1H), 1,10 (s, 9H).

### Beispiel 34: (2R/4S)-2-Amino-1-[4-(4-amino-5-phenyl-pyrrolo[2,3-d]pyrimidin-7-yl)-2-hydroxymethyl-pyrrolidin-1-yl]-3-methyl-pentan-1-on-dihydrochlorid

¹H-NMR (DMSO-d₆, ppm): 8,48 (s, 1H), 8,30 (s, 2H), 7,80 (s, 1H), 7,50 (m, 5H), 5,70 (m, 1H), 4,38 (m, 1H), 4,20 (m, 1H), 3,90 (m, 1H), 3,60 (m, 2H), 3,50 (m, 1H), 3,40 (m, 1H), 2,70 - 2,52 (m, 2H), 1,40 (m, 1H), 1,10 (t, 3H), 0,80 - 0,68 (m, 5H).

### Beispiel 35: (2R/4R)-4-(4-Amino-5-phenyl-pyrrolo[2,3-d]pyrimidin-7-yl)-pyrrolidin-1,2-dicarbonsäure-1-tert.-butylester-2-ethylester

Wird aus (*2R*/*4S*)-4-(Toluyl-4-sulfonyloxy)-pyrrolidin-1,2-dicarbonsäure-1-*tert*.-butylester-2-ethylester und aus 5-Phenyl-7H-pyrrolo[2,3-dpyrimidin-4-yl-amin (Beispiel 1) analog Beispiel 16 hergestellt. ¹H-NMR (CDCl₃, ppm): 8,30 (s, 1H), 7,50 - 7,35 (m, 5H), 7,10 (s, 1H), 5,52 (m, 1H), 5,20 (s, 2H), 4,45 (m, 1H), 4,30 -4,08 (m, 3H), 3,80 (m, 1H), 2,90 (m, 1H), 2,35 (m, 1H), 1,48 (s, 9H), 1,30 (t, 3H).

### Beispiel 36: (2R/4R)-4-(4-Amino-5-phenyl-pyrrolo[2,3-d]pyrimidin-7-yl)-pyrrolidin-2-carbonsäure-ethylester

Wird aus (*2R*/*4R*)-4-(4-Amino-5-phenyl-pyrrolo[2,3-d]pyrimidin-7-yl)-pyrrolidin-1,2-dicarbonsäure-1-*tert*.-butyl-ester-2-ethylester (Beispiel 35) analog Beispiel 17 hergestellt. ¹H-NMR (CD₃OD, ppm): 8,40 (s, 1H), 7,70 (s, 1H), 7,50 - 7,30 (m, 5H), 5,70 (m, 1H), 4,80 (m, 1H), 4,35 (q, 2H), 4,02 - 3,90 (m, 2H), 3,15 (m, 1H), 2,70 (m, 1H), 1,27 (t, 3H).

### Beispiel 37: (2R/4R)-4-(4-Amino-5-phenyl-pyrrolo[2,3-d]pyrimidin-7-yl)-pyrrolidin-2-hydroxymethyl-pyrrolidin-1-carbonsäure-tert.-butylester

Wird aus (*2R*/*4R*)-4-(4-Amino-5-phenyl-pyrrolo[2,3-d]pyrimidin-7-yl)-pyrrolidin-1,2-dicarbonsäure-1-*tert*.-butyl-ester-2-ethylester (Beispiel 35) analog Beispiel 18 hergestellt. ¹H-NMR (CDCl₃, ppm): 8,30 (s, 1H), 7,50 - 7,35 (m, 5H), 7,02 (s, 1H), 5,30 (m, 1H), 5,17 (s, 2H), 4,20 (m, 2H), 3,85 (m, 1H), 3,75 (m, 1H), 3,60 (m, 1H), 2,65 (m, 1H), 2,20 (m, 1H), 1,48 (s, 9H).

### Beispiel 38: (2R/4R)-[4-(4-Amino-5-phenyl-pyrrolo[2,3-d]pyrimidin-7-yl)-pyrrolidin-2-yl]-methanol

Wird aus (*2R*/*4R*)-4-(4-Amino-5-phenyl-pyrrolo[2,3-d]pyrimidin-7-yl)-pyrrolidin-2-hydroxymethyl-pyrrolidin-1-carbonsäure-*tert*.-butylester (Beispiel 37) analog Beispiel 19 hergestellt. ¹H-NMR (CD₃OD, ppm): 8,42 (s, 1H), 7,81 (s, 1H), 7,52 - 7,40 (m, 5H), 5,80 (m, 1H), 4,10 - 3,80 (m, 5H), 2,70 (m, 1H), 2,45 (m, 1H).

### Beispiel 39: (2R/4R)-4-[4-Amino-5-(4-hydroxy-phenyl)-pyrrolo[2,3-d]pyrimidin-7-yl]-pyrrolidin-1,2-dicarbonsäure1-tert.butylester-2-ethylester

Wird aus (*2R*/*4S*)-4-(Toluyl-4-sulfonyloxy)-pyrrolidin-1,2-dicarbonsäure-1-*tert*.-butylester-2-ethylester und aus 5-(4-Benzyloxy-phenyl)-7H-pyrrolo[2,3-d]pyrimidin-4-yl-amin (Beispiel 3) analog Beispiel 20 hergestellt. ¹H-NMR (CDCl₃, ppm): 8,30 (s, 1H), 7,3 (d, 2H), 7,03 (s, 1H), 6,92 (d, 2H), 5,49 (m, 1H), 5,19 (s, 2H), 4,45 (m, 1H), 4,28 - 4,02 (m, 3H), 3,75 (m, 1H), 2,90 (m, 1 H), 2,35 (m, 1H), 1,48 (s, 9H), 1,22 (t, 3H).

### Beispiel 40: (2R/4R)-4-[4-Amino-5-(4-hydroxy-phenyl)-pyrrolo[2,3-d]pyrimidin-7-yl-pyrrolidin-2-carbonsäure-ethylester

Wird aus (*2R*/*4R*)-4-[4-Amino-5-(4-hydroxy-phenyl)-pyrrolo[2,3-d]pyrimidin-7-yl]-pyrrolidin-1,2-dicarbonsäure-1-*tert*.-butylester-2-ethylester (Beispiel 39) analog Beispiel 21 hergestellt. ¹H-NMR (CD₃OD, ppm): 8,32 (s, 1H), 7,53 (s, 1H), 7,30 (d, 2H), 6,92 (d, 2H), 5,65 (m, 1H), 4,72 (m, 1H), 4,38 (m, 2H), 4,02 - 3,88 (m, 2H), 3,15 (m, 1H), 2,78 (m, 1H), 1,32 (t, 3H).

### Beispiel 41: (2R,4R)-4-[4-Amino-5-(4-hydroxy-phenyl)-pyrrolo[2,3-d]pyrimidin-7-yl-2-hydroxymethyl-pyrrolidin-1-carbonsäure-tert.-butylester

Wird aus (*2R*/*4R*)-4-[4-Amino-5-(4-benzyloxy-phenyl)-pyrrolo[2,3-d]pyrimidin-7-yl]-pyrrolidin-1,2-dicarbonsäure-1-*tert*.-butylester-2-ethylester analog Beispiel 22 hergestellt. ¹H-NMR (CD₃OD, ppm): 8,12 (s, 1H), 7,32 (d, 2H), 7,30 (s, 1H), 6,98 (d, 2H), 5,22 (m, 1H), 4,15 (m, 1H), 4,03 (m, 1H), 3,85 (m, 1H), 3,70 (m, 1H), 3,50 (m, 1H), 2,75 (m, 1H), 2,40 (m, 1H), 1,50 (s, 9H).

### Beispiel 42: (2R/4R)-4-[4-Amino-7-(5-hydroxymethyl)-pyrrolidin-3-yl)-7H-pyrrolo[2,3-d]pyrimidin-5-yl]-phenol

Wird aus (*2R*/*4R*)-4-[4-Amino-5-(4-benzyloxy-phenyl)-pyrrolo[2,3-d]pyrimidin-7-yl-2-hydroxymethyl-pyrrolidin-1-carbonsäure-*tert*.-butylester analog Beispiel 23 hergestellt. ¹H-NMR (CD₃OD, ppm): 8,32 (s, 1H), 7,62 (s, 1H), 7,30 (d, 2H), 6,92 (d, 2H), 5,65 (m, 1H), 4,02 - 3,80 (m, 2H), 2,80 (m, 1H), 2,45 (m, 1H).

### Beispiel 43: (2R/4R)-4-(4-Amino-5-phenyl-pyrrolo[2,3-d]pyrimidin-7-yl)-1-(2,2-dimethylpropionyl)-pyrrolidin-2-carbonsäureethylester

Wird aus (*2R*/*4R*)-4-(4-Benzyloxycarbonylamino-5-phenyl-pyrrolo[2,3-d]pyrimidin-7-yl)-1-(2,2-dimethyl-propionyl)-pyrrolidin-2-carbonsäureethylester analog Beispiel 24 hergestellt. ¹H-NMR (CDCl₃, ppm): 8,22 (s, 1H), 7,60 - 7,40 (m, 5H), 7,33 (s, 1H), 5,52 (m, 1H), 4,68 (t, 1H), 4,50 (m, 1H), 4,20 (q, 2H), 3,98 (t, 1H), 2,90 (m, 1H), 2,31 (m, 1H), 1,30 (m, 12H).

### Beispiel 44: (2R/4R)-1-[4-(4-Amino-5-phenyl-pyrrolo[2,3-d]pyrimidin-7-yl)-2-hydroxymethylpyrrolidin-1-yl]-2,2-dimethyl-propan-1-on

Wird aus (*2R*/*4R*)-{7-[1-(2,2-Dimethyl-propionyl)-5-hydroxymethyl-pyrrolidin-3-yl]-5-phenyl-*7H*-pyrrolo[2,3-d]pyrimidin-4-yl}-carbaminsäure-benzylester analog Beispiel 30 hergestellt. ¹H-NMR (CDCl₃, ppm): 8,3 (s, 1H), 7,52 - 7,40 (m, 5H), 7,05 (s, 1H), 2H), 5,30 (s, 2H), 5,20 (m, 1H), 5,30 (s(breit), 2H), 4,50 (m, 2H), 3,90 (m, 1H), 3,75 -3,65 (m, 2H), 2,70 (m, 1H), 2,20 (m, 1H), 1,30 (s, 9H).

### Beispiel 45: 3-(4-Amino-5-phenyl-pyrrolo[2,3-d]pyrimidin-7-yl)-pyrrolidin-1-carbonsäure-tert.butylester

Wird aus 5-Phenyl-*7H*-pyrrolo[2,3-d]pyrimidin-4-yl-amin (Beispiel 1) und 3-(Toluyl-4-sulfonyloxy)-pyrrolidin-1-carbonsäure-*tert*.-butylester *[CAS Reg.-Nr: 103057-45-0]* analog Beispiel 16 hergestellt, ¹H-NMR (CDCl₃, ppm): 8,32 (s, 1H), 7,51-7,35 (m 5H), 6,99 (s, 1H), 5,50 (m, 1H), 5,18 (s, 2H), 3,98 - 3,40 (m, 4H), 2,49 (m, 1H), 2,25 (m, 1H), 1,45 (s, 9H).

### Beispiel 46: 5-Phenyl-7-pyrrolidin-3-yl-7H-pyrrolo[2,3-d]pyrimidin-4-yl-amin

Wird aus 3-(4-Amino-5-phenyl-pyrrolo[2,3-d]pyrimidin-7-yl)-pyrrolidin-1-carbonsäure-*tert*.butylester (Beispiel 45) analog Beispiel 17 hergestellt. ¹H-NMR (DMSO-d₆, ppm): 8,58 (s, 1H), 8,10 (s, 1H), 7,58 - 7,38 (m, 5H), 5,60 (m, 1H), 3,86 - 3,30 (m, 4H), 2,52 (m, 1H), 2,38 (m, 1H).

### Beispiel 47: (2S/4R)-2-(4-Amino-5-phenyl-pyrrolo[2,3-d]pyrimidin-7-ylmethyl)-4-hydroxy-pyrrolidin-1-carbonsäure-tert.-butylester

Wird aus 5-Phenyl-7H-pyrrolo[2,3-d]pyrimidin-4-yl-amin (Beispiel 1) und (2S/4R)-4-Hydroxy-2-(toluyl-4-sulfonyloxymethyl)-pyrrolidin-1-carbonsäure-*tert*.-butylester analog Beispiel 16 hergestellt. ¹H-NMR (CDCl₃, ppm): 8,30 (s, 1H), 7,50-7,38 (m, 5H), 6,92 (s, 1H), 5,12 (s, 2H), 4,60 - 4,32 (m, 4H), 4,10 (m, 1H), 3,60 (m, 1H), 3,40 (m, 1H), 1,49 (s, 9H).

(2S/4R)-4-Hydroxy-2-(toluyl-4-sulfonyloxymethyl)-pyrrolidin-1-carbonsäure-*tert*.-butylester wird nach T. Nakamura et al., *J.Org. Chem.* (1992), **57**, 3783 - 3789 hergestellt.

### Beispiel 48: (3R/5S)-5-(4-Amino-5-phenyl-pyrrolo[2,3-d]pyrimidin-7-ylmethyl)-pyrrolidin-3-ol

Wird aus (2S/4R)-2-(4-Amino-5-phenyl-pyrrolo[2,3-d]pyrimidin-7-ylmethyl)-4-hydroxy-pyrrolidin-1-carbonsäure-*tert*.-butylester (Beispiel 47) analog Beispiel 17 hergestellt. ¹H-NMR (DMSO-d₆, ppm): 8,58 (s, 1H), 7,95 (s, 1H), 7,55 (m, 5H), 4,75 (m, 2H), 4,48 (m, 1H), 4,75 (m, 1H), 3,44 (m, 1H), 3,0 (m, 1H), 2,05 (m, 1H), 1,82 (m, 1H).

### Beipiel 49: (2R/4R)-2-(4-Amino-5-phenyl-pyrrolo[2,3-d]pyrimidin-7-ylmethyl)-4-hydroxy-pyrrolidin-1-carbonsäure-tert.-butylester

Wird aus 5-Phenyl-*7H*-pyrrolo[2,3-d]pyrimidin-4-yl-amin (Beispiel 1) und (*2R*/*4R*)-4-(*tert*.-Butyl-dimethyl-silanyloxy)-2-(toluyl-4-sulfonyloxymethyl)-pyrrolidin-1-carbonsäure-*tert*.butylester analog Beispiel 16 hergestellt. ¹H-NMR (CDCl₃, ppm): 8,31 (s, 1H), 7,52 - 7,32 (m, 5H), 7,10 (s, 1H), 5,22 (s, 2H), 4,80 (m, 1H), 4,55 (m, 2H), 4,30 (m, 2H), 3,60 (m, 1H), 3,48 (m, 1 H), 1,49 (s, 9H).

(*2R*/*4R*)-4-Hydroxy-2-(toluyl-4-sulfonyloxymethyl)-pyrrolidin-1-carbonsäure-*tert*.-butylester *[CAS-Reg.-Nr: 141850-54-6]* wird nach T. Nakamura et al., J.Org. Chem. 1992, 57, 3783 - 3789 hergestellt. Durch Umsetzung dieser Verbindung mit tert.-Butyldimethylsilylchlorid stellt man(*2R*/*4R*)-4-(*tert*.-Butyl-dimethyl-silanyloxy)-2-(toluyl-4-sulfonyloxymethyl)-pyrrolidin-1-carbonsäure-*tert*.-butylester her.

### Beispiel 50: (3R/5R)-5-(4-Amino-5-phenyl-pyrrolo[2,3-d]pyrimidin-7-ylmethyl)-pyrrolidin-3-ol

Wird aus (*2R*/*4R*)-2-(4-Amino-5-phenyl-pyrrolo[2,3-d]pyrimidin-7-ylmethyl)-4-hydroxy-pyrrolidin-1-carbonsäure-tert.-butylester (Beispiel 49) analog Beispiel 17 hergestellt. ¹H-NMR (DMSO-d₆, ppm): 8,60 (s, 1H), 7,75 (s, 1H), 7,55 -7,36 (m, 5H), 4,70 (m, 2H), 4,41 (m, 1H), 4,10 (m, 1H), 3,23 (m, 2H), 2,80 (m, 1 H), 1,80 (m, 1H).

Analog Beispiel 16 werden ausgehend von 5-Phenyl-*7H*-pyrrolo[2,3-d]pyrimidin-4-yl-amin (Beispiel 1) und 4-(Toluyl-4-sulfonyloxy)-piperidin-1,2-dicarbonsäure-1-*tert*.-butylester-2-ethylester, 4-(Toluyl-4-sulfonyloxy)-piperidin-1-carbonsäure-*tert*.-butylester oder 3-(Toluyl-4-sulfonyloxy)-piperidin-1-carbonsäure-*tert*.-butylester die Beispiele 51 bis 54 hergestellt.

### Beispiel 51: 3-(4-Amino-5-phenyl-pyrrolo[2,3-d]pyrimidin-7-yl)-piperidin-1,2-dicarbonsäure-1-tert.-butylester-2-ethylester

¹H-NMR (CD₃OD, ppm): 8,20 (s, 1H), 7,58 (s, 1H), 7,56 - 7,24 (m, 5H), 5,5 (m, 1H), 5,05 (m, 1H), 4,10 (m, 1H), 3,98 (m, 1H), 3,90 (m, 1H), 3,30 (m, 1H), 2,40 (m, 1H), 2,20 (m, 1H), 2,05 (m, 1H), 1,80 (m, 1H), 1,45 (d, 9H), 0,95 (m, 3H).

### Beispiel 52: 4-(4-Amino-5-phenyl-pyrrolo[2,3-d]pyrimidin-7-yl)-piperidin-1,2-dicarbonsäure-1-tert.-butylester-2-ethylester

¹H-NMR (CD₃OD, ppm): 8,17 (s, 1H), 7,62 (s, 1H), 7,58 - 7,40 (m, 5H), 5,08 (m, 1H), 4,95 (m, 1H), 4,35 - 4,20 (m, 3H), 3,20 (m, 1H), 2,60 (m, 1H), 2,40 (m, 1H), 2,20 (m, 1H), 2,08 (m, 1H), 1,45 (d, 9H), 1,30 (m, 3H).

### Beispiel 53: 4-(4-Amino-5-phenyl-pyrrolo[2,3-d]pyrimidin-7-yl)-piperidin-1-carbonsäure-tert.butylester

¹H-NMR (CDCl₃, ppm): 8,31 (s, 1H), 7,50 - 7,30 (m, 5H), 7,01 (s, 1H), 5,15 (s, 2H), 4,90 (m, 1H), 4,35 (m, 2H), 3,0 (m, 2H), 2,10 (m, 2H), 1,90 (m, 2H), 1,50 (s, 9H).

### Beispiel 54: 3-(4-Amino-5-phenyl-pyrrolo[2,3-d]pyrimidin-7-yl)-piperidin-1-carbonsäure-tert.butylester

¹H-NMR (CDCl₃, ppm):8,31 (s, 1H), 7,52 - 7,32 (m, 5H), 7,02 (s, 1H), 5,20 (s, 2H), 4,80 (m, 1H), 4,30 (m, 1H), 4,00 (m, 1H), 3,30 (m, 1H), 3,00 (m, 1H), 2,23 (m, 1H), 2,05 (m, 1H), 1,49 (s, 9H).

### Beispiel 55: 3-(4-Amino-5-phenyl-pyrrolo[2,3-d]pyrimidin-7-yl)-piperidin-2-carbonsäure-ethyl-ester-dihydrochlorid

Wird ausgehend von 3-(4-Amino-5-phenyl-pyrrolo[2,3-d]pyrimidin-7-yl)-piperidin-1,2-dicarbonsäure-1-*tert*.-butylester-2-ethylester (Beispiel 51) analog Beispiel 17 hergestellt. ¹H-NMR (CD₃OD, ppm): 8,18 (s, 1H), 7,62 (s, 1H), 7,52 - 7,40 (m, 5H), 5,00 (m, 1H), 4,78 (m, 1H), 4,50 - 4,37 (m, 2H), 3,60 (m, 2H), 3,60 (m, 2H), 2,78 (m, 2H), 2,48 (m, 1H), 2,28 (m, 1H), 1,20 (t, 3H).

### Beispiel 56: 4-(4-Amino-5-phenyl-pyrrolo[2,3-d]pyrimidin-7-yl)piperidin-2-carbonsäure-ethylester-dihydrochlorid

Wird ausgehend von 4-(4-Amino-5-phenyl-pyrrolo[2,3-d]pyrimidin-7-yl)-piperidin-1,2-dicarbonsäure-1-*tert*.-butylester-2-ethylester (Beispiel 52) analog Beispiel 17 hergestellt. ¹H-NMR (CD₃OD, ppm): 8,41 (s, 1H), 7,60 (s, 1H), 7,52 -7,42 (m, 5H), 5,50 (m, 1H), 5,00 (d, 1H), 4,18 - 4,02 (m, 2H), 3,80 (m, 1H), 3,38 (m, 1H), 2,28 (m, 2H), 2,12 (m, 1H), 2,00 (m, 1H), 0,98 (t, 3H).

### Beispiel 57: (5-Phenyl-7-piperidin-4-yl-7H-pyrrolo[2,3-d]pyrimidin)-4-yl-amin-dihydrochlorid

Wird ausgehend von 4-(4-Amino-5-phenyl-pyrrolo[2,3-d]pyrimidin-7-yl)-piperidin-1-carbonsäure-*tert*.-butylester (Beispiel 53) analog Beispiel 17 hergestellt, ¹H-NMR (DMSO-d₆, ppm): 8,51 (s, 1H), 7,68 (s, 1H), 7,51 -7,40 (m, 5H), 5,00 (m, 1H), 3,48 (m, 2H), 3,18 (m, 2H), 2,40 (m, 2H), 2,18 (m, 2H).

### Beisplel 58: (5-Phenyl-7-piperidin-3-yl-7H-pyrrolo[2,3-d]pyrimidin)-4-yl-amin-dihydrochlorid

Wird ausgehend von 3-(4-Amino-5-phenyl-pyrrolo[2,3-d]pyrimidin-7-yl)-piperidin-1-carbonsäure-tert.-butylester (Beispiel 54) analog Beispiel 17 hergestellt, ¹H-NMR (DMSO-d₆, ppm): 8,55 (s, 1H),7,90 (s, 1H), 7,50 - 7,40 (m, 5H), 5,20 (m, 1H), 3,51 - 3,30 (m, 3H), 2,88 (m, 1H), 2,30 - 1,89 (m, 4H).

Analog Beispiel 16 werden ausgehend von 5-(3-Methoxy-phenyl)-*7H*-pyrrolo[2,3-d]pyrimidin-4-yl-amin resp. 5-(4-Methoxy-phenyl)-*7H*-pyrrolo[2,3-d]pyrimidin-4-yl-amin die Beispiele 59-62 hergestellt

### Beispiel 59: 4-[4-Amino-5-(4-methoxy-phenyl)-pyrrolo[2,3-d]pyrimidin-7-yl]-pyrrolidin-1,2-dicarbonsäure-1-tert.-butylester-2-ethylester

### Beispiel 60: 4-[4-Amino-5-(3-methoxy-phenyl)-pyrrolo[2,3-d]pyrimidin-7-yl]-pyrrolidin-1,2-dicarbonsäure-1-tert.-butylester-2-ethylester

### Beispiel 61: 3-[4-Amino-5-(4-methoxy-phenyl)-pyrrolo[2,3-d]pyrimidin-7-yl]-pyrrolidin-1-carbonsäure-tert.-butylester

¹H-NMR (CDCl₃, ppm): 8,32 (s, 1H), 7,40 (m, 1H), 7,10 - 6,86 (m, 3H), 5,50 (m, 1H), 5,30 (s, 2H), 4,01 - 3,82 (m, 2H), 3,69 - 3,48 (m, 2H), 2,45 (m, 1H), 2,30 (m, 1H), 1,50 (s, 9H).

### Beispiel 62: 3-[4-Amino-5-(3-methoxy-phenyl)-pyrrolo[2,3-d]pyrimidin-7-yl]-pyrrolidin-1-carbonsäure-tert.-butylester

¹H-NMR (CDCl₃, ppm): 8,31 (s, 1H), 7,38 (d, 2H), 6,99 (d, 2H), 6,90 (s, 1H), 5,47 (m, 1H), 5,24 (s, 2H), 3,95 - 3,81 (m, 2H), 3,72 -3,51 (m, 2H), 2,42 (m, 1H), 2,27 (m, 1 H), 1,48 (s, 9H).

Analog Beispiel 20/21 werden ausgehend von 5-(4-Benzyloxy-phenyl)-*7H*-pyrrolo[2,3-d]-pyrimidin-4-yl-amin resp. 5-(3-Benzyloxy-phenyl)-7H-pyrrolo[2,3-d]pyrimidin-4-yl-amin und 3-(Toluol-4-sulfonylmethyl)-pyrrolidin-1-carbonsäure-*tert*.-butylester die Beispiele 63 und 64 hergestellt.

### Beispiel 63: 5-(4-Benzyloxy-phenyl)-7-pyrrolidin-3-yl-7H-pyrrolo[2,3-d]pyrimidin-4-ylamindihydrochlorid

¹H-NMR (DMSO-d₆, ppm): 8,52 (s, 1H), 7,92 (s, 1H), 7,52 - 7,32 (m, 7H), 7,18 (d, 2H), 5,10 (m, 1H), 5,18 (s, 2H), 3,76 (m, 1H), 3,57 (m, 2H), 3,38 (m, 1H), 2,58 (m, 1H), 2,34 (m, 1H).

### Beispiel 64: 5-(3-Benzyloxy-phenyl)-7-pyrrolidin-3-yl-7H-pyrrolo[2,3-d]pyrimidin-4-ylamindihydrochlorid

¹H-NMR (DMSO-d₆, ppm): 8,52 (s, 1H), 8,05 (s, 1H), 7,50 - 7,28 (m, 6H), 7,18 -7,02 (m, 3H), 5,55 (m,1H), 5,15 (s, 2H), 3,80 (m, 1H), 3,61 (m, 2H), 3,38 (m, 1H), 2,56 (m, 1H), 2,37 (m, 1H).

Analog Beispiel 20/20a wird ausgehend von 5-(4-Benzyloxy-phenyl)-*7H*-pyrrolo[2,3-d]-pyrimidin-4-yl-amin und 3-(Toluol-4-sulfonylmethyl)-pyrrolidin-1-carbonsäure-tert.-butylester das Beispiel 65 hergestellt.

### Beispiel 65: 3-[4-Amino-5-(4-hydroxy-phenyl)-pyrrolo[2,3-d]pyrimidin-7-yl]-pyrrolidin-1-carbonsäure-tert.-butylester

¹H-NMR (DMSO-d₆, ppm): 9,52 (s, 1H), 8,27 (s,1H), 7,28 (d, 2H), 6,89 (d, 2H), 6,09 (s; breit), 2H), 5,28 (m,1H), 3,80 (m, 1H), 3,62 - 3,40 (m, 3H), 2,38 (m, 2H), 1,42 (s, 9H).

Analog Beispiel 21 wird ausgehend von 3-[4-Amino-5-(4-hydroxy-phenyl)-pyrrolo[2,3-d]-pyrimidin-7-yl]-pyrrolidin-1-carbonsäure-*tert*.-butylester (Beispiel 65) Beispiel 66 hergestellt.

### Beispiel 66: 4-(4-Amino-7-pyrrolidin-3-yl-7H-pyrrolo[2,3-d]pyrimidin-5-yl)-phenol-dihydrochlorid

¹H-NMR (DMSO-d₆, ppm): 9,99 (s; breit), 1H), 9,76 (s; breit), 1H), 8,50 (s, 1H), 7,90 (s, 1H), 7,30 (d, 2H), 6,90 (d, 2H), 5,58 (m, 1H), 3,72 (m,1H), 3,49 (m, 3H), 3,31 (m, 1H), 2,52 (m, 1H), 2,30 (m, 1 H).

Analog Beispiel 24 werden ausgehend von 4-[4-Amino-5-(4-methoxy-phenyl)-pyrrolo[2,3-d]-pyrimidin-7-yl]-pyrrolidin-1,2-dicarbonsäure-1-*tert*.-butylester-2-ethylester (Beispiel 59) die Beispiele 67 - 70 hergestellt.

### Beispiel 67: 4-[4-Amino-5-(4-methoxy-phenyl)-pyrrolo[2,3-d]pyrimidin-7-yl]-1-(3,3-dimethylbutyryl)-pyrrolidin-2-carbonsäure-ethylester

### Beispiel 68: 4-[4-Amino-5-(3-methoxy-phenyl)-pyrrolo[2,3-d]pyrimidin-7-yl]-1-(3.3-dimethylbutyryl)-pyrrolidin-2-carbonsäure-ethylester

### Beispiel 69: 4-[4-Amino-5-(4-methoxy-phenyl)-pyrrolo[2,3-d]pyrimidin-7-yl]-1-(2-amino-3-methyl-pentanoyl)-pyrrolidin-2-carbonsäure-ethylester

### Beispiel 70: 4-[4-Amino-5-(3-methoxy-phenyl)-pyrrolo[2,3-d]pyrimidin-7-yl]-1-(2-amino-3-methyl-pentanoyl)-pyrrolidin-2-carbonsäure-ethylester

Analog Beispiel 30 werden ausgehend von 4-[4-Amino-5-(4-methoxy-phenyl)-pyrrolo[2,3-d]-pyrimidin-7-yl]-pyrrolidin-1,2-dicarbonsäure-1-*tert*.-butylester-2-ethylester (Beispiel 59, resp. Beispiel 60) die Beispiele 71 - 72 hergestellt.

### Beispiel 71: 1-{4-[4-Amino-5-(4-methoxy-phenyl)-pyrrolo[2,3-d]pyrimidin-7-yl]-2-hydroxymethyl-pyrrolidin1-yl}-3,3-dimethyl-butan-1-on

### Beispiel 72: 1-{4-[4-Amino-5-(3-methoxy-phenyl)-pyrrolo[2,3-d]pyrimidin-7-yl]-2-hydroxymethyl-pyrrolidin1-yl}-3.3-dimethyl-butan-1 -on

Analog Beispiel 17 werden die Beispiele 73 - 76 hergestellt.

### Beispiel 73: 4-[4-Amino-5-(4-methoxy-phenyl)-pyrrolo[2,3-d]pyrimidin-7-yl]-pyrrolidin-2-carbonsäure-ethylester

Wird ausgehend von 4-[4-Amino-5-(4-methoxy-phenyl)-pyrrolo[2,3-d]pyrimidin-7-yl]-pyrrolidin-1,2-dicarbonsäure-1-*tert*.-butylester-2-ethylester (Beispiel 59) analog Beispiel 17 hergestellt.

### Beispiel 74: 4-[4-Amino-5-(3-methoxy-phenyl)-pyrrolo[2,3-d]pyrimidin-7-yl]-pyrrolidin-2-carbonsäure-ethylester

Wird ausgehend von 4-[4-Amino-5-(3-methoxy-phenyl)-pyrrolo[2,3-d]pyrimidin-7-yl]-pyrrolidin-1,2-dicarbonsäure-1-*tert*.-butylester-2-ethylester (Beispiel 60) analog Beispiel 17 hergestellt.

### Beispiel 75: 5-(4-Methoxy-phenyl)-7-pyrrolidin-3-yl-7H-pyrrolo[2,3-d]pyrimidin-4-yl-amin-dihydrochlorid

Wird ausgehend von 3-[4-Amino-5-(4-methoxy-phenyl)-pyrrolo[2,3-d]pyrimidin-7-yl]-pyrrolidin-1-carbonsäure-*tert*.-butylester (Beispiel 61) analog Beispiel 17 hergestellt. ¹H-NMR (DMSO-d₆, ppm): 10,0 (s; breit), 1H), 9,80 (s (breit), 1H), 8,52 (s, 1H), 7,98 (s, 1H), 7,42 (d, 2H), 7,08 (d, 2H), 5,10 (m, 1H), 3,75 (m, 1H), 3,60 (m, 2H), 3,40 (m, 1H), 2,55 (m, 1H), 2,32 (m, 1H).

### Beispiel 76: 5-(3-Methoxy-phenyl)-7-pyrrolidin-3-yl-7H-pyrrolo[2,3-d]pyrimidin-4-yl-amin-dihydrochlorid

Wird ausgehend von 3-[4-Amino-5-(3-methoxy-phenyl)-pyrrolo[2,3-d]pyrimidin-7-yl]-pyrrolidin-1-carbonsäure-*tert*.-butylester (Beispiel 62) analog Beispiel 17 hergestellt. ¹H-NMR (DMSO-d₆, ppm): 10,10 (s , 1H), 9,80 (s, 1H), 8,52 (s, 1H), 8,10 (s, 1H), 7,42 (m, 1H), 7,10 (m, 2H), 6,99 (m, 1H), 5,10 (m, 1H), 3,75 (m, 1H), 3,54 (m, 2H), 3,38 (m, 1H), 2,52 (m, 1H), 2,32 (m, 1H).

Analog Beispiel 23 werden ausgehend von 5-(4-Benzyloxy-phenyl)-*7H*-pyrrolo[2,3-d]pyrimidin-4-yl-amin resp. 5-(3-Benzyloxy-phenyl)-*7H*-pyrrolo[2,3-d]pyrimidin-4-yl-amin und 3-(Toluyl-4-sulfonyloxy)-pyrrolidin-1-carbonsäure-*tert*.-butylester die Beispiele 77 und 78 hergestellt.

### Beispiel 77: 4-(4-Amino-7-pyrrolidin-3-yl-7H-pyrrolo[2,3-d]pyrimidin-5-yl)-phenol

### Beispiel 78: 3-(4-Amino-7-pyrrolidin-3-yl-7H-pyrrolo[2,3-d]pyrimidin-5-yl)-phenol

Analog Beispiel 45 werden ausgehend von 5-Phenyl-*7H*-pyrrolo[2,3-d]pyrimidin-4-yl-amin und den entsprechenden 1-substituierten 3-(Toluyl-4-sulfonyloxy)-pyrrolidinen die Beispiele 79 - 3 hergestellt:

### Beispiel 79: 2-{2-[3-(4-Amino-5-phenyl-pyrrolo[2,3-d]pyrimidin-7-yl)-pyrrolidin-1-yl]-ethylamino}-ethanol

### Beispiel 80: 7-[1-(2-Morpholin-4-yl-ethyl)-pyrrolidin-3-yl]-5-phenyl-7H-pyrrolo[2,3-d]pyrimidin-4-yl-amin

### Beispiel 81: 7-[1-(2-Imidazol-1-yl-ethyl)-pyrrolidin-3-yl]-5-phenyl-7H-pyrrolo[2,3-d]pyrimidin-4-yl-amin

### Beispiel 82: 2-[3-(4-Amino-5-phenyl-pyrrolo[2,3-d]pyrimidin-7-yl)-pyrrolidin-1-yl]-ethanol

### Beispiel 83: 7-[1-(2-Methoxy-ethyl)-pyrrolidin-3-yl]-5-phenyl-7H-pyrrolo[2,3-d]pyrimidin-4-ylamin

Analog Beispiel 20 werden ausgehend von 5-(4-Benzyloxy-phenyl-*7H*-pyrrolo[2,3-d]pyrimidin-4-yl-amin resp. 5-(3-Benzyloxy-phenyl-*7H*-pyrrolo[2,3-d]pyrimidin-4-yl-amin und den entsprechenden 1-substituierten 3-(Toluyl-4-sulfonyloxy)-pyrrolidinen die Beispiele 84 - 99 hergestellt:

### Beispiel 84: 4-(4-Amino-7-{1-[2-(2-hydroxy-ethylamino)-ethyl]-pyrrolidin-3-yl)-7H-pyrrolo-[2,3-d]pyrimidin-5-yl)-phenol

### Beispiel 85: 3-(4-Amino-7-{1-[2-(2-hydroxy-ethylamino)-ethyl]-pyrrolidin-3-yl}-7H-pyrrolo-[2,3-d]pyrimidin-5-yl)-phenol

### Beispiel 86: 4-{4-Amino-7-[1-(2-morpholin-4-yl-ethyl)-pyrrolidin-3-yl]-7H-pyrrolo[2,3-d]pyrimidin-5-yl}-phenol

### Belspiel 87: 3-{4-Amino-7-[1-(2-morpholin-4-yl-ethyl)-pyrrolidin-3-yl]-7H-pyrrolo[2,3-d]pyrimidin-5-yl}-phenol

### Beispiel 88: 4-{4-Amino-7-[1-(2-imidazol-1-yl-ethyl)-pyrrolidin-3-yl]-7H-pyrrolo[2,3-d]pyrimidin-5-yl}-phenol

### Beispiel 89: 3-{4-Amino-7-[1-(2-imidazol-1-yl-ethyl)-pyrrolidin-3-yl]-7H-pyrrolo[2,3-d]pyrimidin-5-yl}-phenol

### Beispiel 90: 4-{4-Amino-7-[1-(2-methoxy-ethyl)-pyrrolidin-3-yl]-7H-pyrrolo[2,3-d]pyrimidin-5-yl}phenol

### Beispiel 91: 3-{4-Amino-7-[1-(2-methoxy-ethyl)-pyrrolidin-3-yl]-7H-pyrrolo[2,3-d]pyrimidin-5-yl}-henol

### Beispiel 92: 4-{4-Amino-7-[1-(2-imidazol-1-yl-ethyl)-pyrrolidin-3-yl]-7H-pyrrolo[2,3-d]pyrimidin-5-yl}-phenol

### Beispiel 93: 3-{4-Amino-7-[1-(2-imidazol-1-yl-ethyl)-pyrrolidin-3-yl}-7H-pyrrolo[2,3-d]pyrimidin-5-yl}-phenol

### Beispiel 94: 4-{4-Amino-7-[1-(2-hydroxy-ethyl)-pyrrolidin-3-yl]-7H-pyrrolo[2,3-d]pyrimidin-5-yl}-phenol

### Beispiel 95: 3-{4-Amino-7-[1-(2-hydroxy-ethyl)-pyrrolidin-3-yl]-7H-pyrrolo[2,3-d]pyrimidin-5-yl}-phenol

### Beispiel 96: {3-[4-Amino-5-(3-benzyloxy-phenyl)-pyrrolo[2,3-d]pyrimidin-7-yl]-pyrrolidin-1-yl)-essigsäure-methylester

¹H-NMR (CDCl₃, ppm): 8,3 (s, 1H), 7,49 - 7,39 (m, 7H), 7,1 (m, 2H), 6,95 (m, 1H), 5,5 (m, 1H), 5,12 (s, 2H), 5,05 (s, 2H), 3,71 (s, 3H), 3,42 (q, 2H), 3,29 - 3,13 (m, 2H), 2,99 (m, 1H), 2,70 -2,51 (m, 2H), 2,08 (m, 1 H).

### Beispiel 97: {3-[4-Amino-5-(3-hydroxy-phenyl)-pyrrolo[2,3-d]pyrimidin-7-yl]-pyrrolidin-1-yl}essigsäure-methylester

¹H-NMR (DMSO-d₆, ppm): 9.59 (s, 1H), 8,12 (s, 1H), 7,28 (t, 1H), 6,82 (m, 2H), 6,72 (d, 1H), 6.10 (s; breit), 2H), 5,11 (m, 1H), 3,62 (s, 3H), 3,45 (q, 2H), 3,16 - 2,82 (m, 3H), 2,60 (m, 1H), 2,42 (m, 1H) 1,95 (m, 1H).

### Beispiel 98: 2-{3-[4-Amino-5-(3-hydroxy-phenyl)-pyrrolo[2,3-d]pyrimidin-7-yl]-pyrrolidin-1-yl)-N,N-dimethyl-acetamid

¹H-NMR (CD₃OD, ppm): 8,12 (s, 1H), 7,53 (s, 1H), 7,29 (m, 1H), 6,96 (m, 2H), 6,80 (m, 1H), 5,41 (m, 1H), 3,50 (m, 1H), 3,30 (m, 1H), 3,20 (m, 1H), 3,10 (s, 3H), 2,93 (s, 3H), 2,88 (m, 1H), 2,59 (m, 2H), 2,09 (m, 1H).

### Beispiel 99: 2-{3-[4-Amino-5-(3-hydroxy-phenyl)-pyrrolo[2,3-d]pyrimidin-7-yl]-pyrrolidin-1-yl)-acetamid

¹H-NMR (DMSO-d₆, ppm): 9.70 (s, 1H), 8,12 (s, 1H), 7,58 (s, 1H), 7,46 (s; breit), 1H), 7,25 (t, 1H), 7,15 (s; breit), 1H), 6,88 (m, 2H), 6,79 (m, 1H), 6,12 (s; breit), 2H), 5,38 ( m, 1H), 3,40 (m, 1H), 2,93 (m, 2H), 2,57 (m, 1H), 2,42 (m, 1H), 2,05 (m, 1H).

Analog Beispiel 45 werden ausgehend von 5-(4-Methoxy-phenyl)-7H-pyrrolo[2,3-d]pyrimidin-4-yl-amin resp. 5-(3-Methoxy-phenyl)-*7H*-pyrrolo[2,3-d]pyrimidin-4-yl-amin und den entsprechenden 1-substituierten 3-(Toluyl-4-sulfonyloxy)-pyrrolidinen die Beispiele 100 - 109 hergestellt.

### Beispiel 100: 2-(2-{3-[4-Amino-5-(4-methoxy-phenyl)-pyrrolo[2,3-d]pyrimidin-7-yl]-pyrrolidin-1-yl}-ethylamino)-ethanol

### Beispiel 101: 2-(2-{3-[4-Amino-5-(3-methoxy-phenyl)-pyrrolo[2,3-d]pyrimidin-7-yl]-pyrrolidin-1-yl}-ethylamino)-ethanol

### Beispiel 102: 5-(4-Methoxy-phenyl)-7-[1-(2-morpholin-4-yl-ethyl)-pyrrolidin-3-yl]-7H-pyrrolo-[2,3-d]pyrimidin-4-yl-amin

### Beispiel 103: 5-(3-Methoxy-phenyl)-7-[1-(2-morpholin-4-yl-ethyl)-pyrrolidin-3-yl]-7H-pyrrolo-[2,3-d]pyrimidin-4-yl-amin

### Beispiel 104: 7-[1-(2-Imidazol-1-yl-ethyl)-pyrrolidin-3-yl]-5-(4-methoxy-phenyl)-7H-pyrrolo-[2,3-d]pyrimidin-4-yl-amin

### Beispiel 105: 7-[1-(2-Imidazol-1-yl-ethyl)-pyrrolidin-3-yl]-5-(3-methoxy-phenyl)-7H-pyrrolo-[2,3-d]pyrimidin-4-yl-amin

### Beispiel 106: 7-[1-(2-Methoxy-ethyl)-pyrrolidin-3-yl]-5-(4-methoxy-phenyl)-7H-pyrrolo[2,3-d]-pyrimidin-4-yl-amin

### Beispiel 107: 7-[1-(2-Methoxy-ethyl)-pyrrolidin-3-yl]-5-(3-methoxy-phenyl)-7H-pyrrolo[2,3-d]-pyrimidin-4-yl-amin

### Beispiel 108: {3-[4-Amino-5-(3-methoxy-phenyl)-pyrrolo[2,3-d]pyrimidin-7-yl]-pyrrolidin-1-yl}essigsäure-methylester

Analog Beispiel 16 werden ausgehend von 5-(4-Methoxy-phenyl)-7H-pyrrolo[2,3-d]pyrimidin-4-yl-amin resp. 5-(3-Methoxy-phenyl)-*7H*-pyrrolo[2,3-d]pyrimidin-4-yl-amin und 4-(Toluyl-4-sulfonyloxy)-piperidin-1,2-dicarbonsäure-1-*tert*.-butylester-2-ethylester die Beispiele 109 und 110 hergestellt.

### Beispiel 109: 4-[4-Amino-5-(4-methoxy-phenyl)-pyrrolo[2,3-d]pyrimidin-7-yl]-piperidin-1,2-dicarbonsäure-1-tert.-butylester-2-ethylester

### Beispiel 110: 4-[4-Amino-5-(3-methoxy-phenyl)-pyrrolo[2,3-d]pyrimidin-7-yl]-piperidin-1,2-dicarbonsäure-1-tert.-butylester-2-ethylester

Analog Beispiel 20 werden ausgehend von 5-(4-Benzyloxy-phenyl)-*7H*-pyrrolo[2,3-d]pyrimidin-4-yl-amin resp. 5-(3-Benzyloxy-phenyl)-*7H*-pyrrolo[2,3-d]pyrimidin-4-yl-amin und 4-(Toluyl-4-sulfonyloxy)-piperidin-1,2-dicarbonsäure-1-*tert*.-butylester-2-ethylester die Beispiele 111 - 115 hergestellt.

### Beispiel 111: 4-[4-Amino-5-(3-benzyloxy-phenyl)-pyrrolo[2,3-d]pyrimidin-7-yl]-piperidin-1-carbonsäure-tert.butylester

¹H-NMR (CDCl₃, ppm): 8,30 (s, 1H), 7,40 (m, 6H), 7,10 - 6,92 (m, 4H), 5,12 (s, 4H), 4,88 (m, 1H), 4,32 (m, 2H), 2,96 (m, 2H), 2,11 (m, 1H), 1,97 - 1,82 (m, 3H), 1,49 (s, 9H).

### Beispiel 112: 4-[4-Amino-5-(4-hydroxy-phenyl)-pyrrolo[2,3-d]pyrimidin-7-yl]-piperidin-1,2-dicarbonsäure-1-tert.-butylester-2-ethylester

### Beispiel 113: 4-[4-Amino-5-(3-hydroxy-phenyl)-pyrrolo[2,3-d]pyrimidin-7-yl]-piperidin-1,2-dicarbonsäure-1-tert.-butylester-2-ethylester

Analog Beispiel 17 werden Beispiele 114 - 117 hergestellt.

### Beispiel 114: 4-[4-Amino-5-(4-methoxy-phenyl)-pyrrolo[2,3-d]pyrimidin-7-yl]-piperidin-2-carbonsäure-ethylester

Wird ausgehend von 4-[4-Amino-5-(4-methoxy-phenyl)-pyrrolo[2,3-d]pyrimidin-7-yl]-piperidin-1,2-dicarbonsäure-1-*tert*.-butylester-2-ethylester (Beispiel 100) analog Beispiel 17 hergestellt.

### Beispiel 115: 4-[4-Amino-5-(3-methoxy-phenyl)-pyrrolo[2,3-d]pyrimidin-7-yl]-piperidin-2-carbonsäure-ethylester

Wird ausgehend von 4-[4-Amino-5-(3-methoxy-phenyl)-pyrrolo[2,3-d]pyrimidin-7-yl]-piperidin-1,2-dicarbonsäure-1-*tert*.-butylester-2-ethylester (Beispiel 110) analog Beispiel 17 hergestellt.

### Beispiel 116: 4-[4-Amino-5-(4-hydroxy-phenyl)-pyrrolo[2,3-d]pyrimidin-7-yl]-piperidin-2-carbonsäure-ethylester

Wird ausgehend von 4-[4-Amino-5-(4-hydroxy-phenyl)-pyrrolo[2,3-d]pyrimidin-7-yl]-piperidin-1,2-dicarbonsäure-1-*tert*.-butylester-2-ethylester (Beispiel 112) analog Beispiel 17 hergestellt.

### Beispiel 117: 4-[4-Amino-5-(3-hydroxy-phenyl)-pyrrolo[2,3-d]pyrimidin-7-yl]-piperidin-2-carbonsäure-ethylester

Wird ausgehend von 4-[4-Amino-5-(3-hydroxy-phenyl)-pyrrolo[2,3-d]pyrimidin-7-yl]-piperidin-1,2-dicarbonsäure-1-*tert*.-butylester-2-ethylester (Beispiel 113) analog Beispiel 17 hergestellt.

Analog Beispiel 16 werden ausgehend von 5-(4-Methoxy-phenyl)-*7H*-pyrrolo[2,3-d]pyrimidin-4-yl-amin resp. 5-(3-Methoxy-phenyl)-*7H*-pyrrolo[2,3-d]pyrimidin-4-yl-amin und 4-(Toluyl-4-sulfonyloxy)-piperidin-1-carbonsäure-*tert*.-butylester die Beispiele 118 und 119 hergestellt.

### Beispiel 118: 4-[4-Amino-5-(4-methoxy-phenyl)-pyrrolo[2,3-d]pyrimidin-7-yl]-piperidin-1-carbonsäure-tert.-butylester

### Beispiel 119: 4-[4-Amino-5-(3-methoxy-phenyl)-pyrrolo[2,3-d]pyrimidin-7-yl]-piperidin-1-carbonsäure-tert.-butylester

¹H-NMR (CDCl₃, ppm): 8,32 (s, 1H), 8,05 (s, 1H), 7,38 (t, 1H), 7,08 - 6,87 (m, 3H), 5,22 (s, 2H), 4,90 (m, 1 H), 4,31 (m, 2H), 3,88 (s, 3H), 2,97 (m, 2H), 2,10 (m, 2H), 1,90 (m, 2H), 1,50 (s, 9H).

Analog Beispiel 16 wird ausgehend von 5-(3-Fluor-phenyl)-*7H*-pyrrolo[2,3-d]pyrimidin-4-ylamin und 4-(Toluyl-4-sulfonyloxy)-piperidin-1-carbonsäure*-tert*.-butylester das Beispiel 120 hergestellt.

### Beispiel 120: 4-[4-Amino-5-(3-fluor-phenyl)-pyrrolo[2,3-d]pyrimidin-7-yl]-piperidin-1-carbonsäure-tert.-putylester

¹H-NMR (CDCl₃, ppm): 8,38 (s, 1H), 7,52 - 7,38 (m, 1H), 7,28 -7,02 (m, 3H), 7,00 (s, 1H), 4,90 (m, 1H), 4,32 (m, 2H), 2,98 (m, 2H), 2,10 (m, 2H), 1,91 (m, 1H), 1,50 (s, 9H).

Analog Beispiel 20 werden ausgehend von 5-(4-Benzyloxy-phenyl)-*7H*-pyrrolo[2,3-d]pyrimidin-4-yl-amin resp. 5-(3-Benzyloxy-phenyl)-*7H*-pyrrolo[2,3-d]pyrimidin-4-yl-amin und 4-(Toluyl-4-sulfonyloxy)-piperidin-1-dicarbonsäure*-tert.*-butylester die Beispiele 121 und 122 hergestellt.

### Beispiel 121: 4-[4-Amino-5-(4-hydroxy-phenyl)-pyrrolo[2,3-d]pyrimidin-7-yl]-piperidin-1-carbonsäure-tert.-butylester

### Beispiel 122: 4-[4-Amino-5-(3-hydroxy-phenyl)-pyrrolo[2,3-d]pyrimidin-7-yl]-piperidin-1-carbonsäure-tert.-butylester

¹H-NMR (DMSO-d₆, ppm): 9,58 (s, 1H), 8,13 (s, 1H), 7,49 (s, 1H), 7,28 (t, 1H), 6,88 (m, 2H), 6,78 (m, 1H), 6,28 (s; breit), 2H), 4,78 (m, 1H), 4,12 (m, 2H), 2,96 (m, 2H), 1,90 (m, 4H), 1,42 (s, 9H).

Analog Beispiel 17 werden Beispiele 123 - 126 hergestellt.

### Beispiel 123: 5-(4-Methoxy-phenyl)-7-piperidin-4-yl-7H-pyrrolo[2,3-d]pyrimidin-4-yl-amin

Wird ausgehend von 4-[4-Amino-5-(4-methoxy-phenyl)-pyrrolo[2,3-d]pyrimidin-7-yl]-piperidin-1-carbonsäure-*tert*.-butylester (Beispiel 118) analog Beispiel 17 hergestellt.

### Beispiel 124: 5-(3-Methoxy-phenyl)-7-piperidin-4-yl-7H-pyrrolo[2,3-d]pyrimidin-4-yl-amin

Wird ausgehend von 4-[4-Amino-5-(3-methoxy-phenyl)-pyrrolo[2,3-d]pyrimidin-7-yl]-piperidin-1-carbonsäure-*tert*.-butylester (Beispiel 119) analog Beispiel 17 hergestellt. ¹H-NMR (DMSO-d₆, ppm): 9,48 (m; breit), 1H), 9,30 (m; breit), 1H), 8,52 (s, 1 H), 7,70 (s, 1H), 7,42 (t, 1H), 7,12 (m, 2H), 6,99 (m, 1H), 5,00 (m, 1H), 3,82 (s, 3H), 3,46 (m, 2H), 3,12 (m, 2H), 2,40 (m, 2H), 2,12 (m, 2H).

### Beispiel 125: 4-(4-Amino-7-piperidin-4-yl-7H-pyrrolo[2,3-d]pyrimidin-5-yl)-phenol

Wird ausgehend von 4-[4-Amino-5-(4-hydroxy-phenyl)-pyrrolo[2,3-d]pyrimidin-7-yl]-piperidin-1-carbonsäure-*tert*.-butylester (Beispiel 121) analog Beispiel 17 hergestellt.

### Beispiel 126: 3-(4-Amino-7-piperidin-4-yl-7H-pyrrolo[2,3-d]pyrimidin-5-yl)-phenol

Wird ausgehend von 4-[4-Amino-5-(3-hydroxy-phenyl)-pyrrolo[2,3-d]pyrimidin-7-yl]-piperidin-1-carbonsäure-*tert*.-butylester (Beispiel 122) analog Beispiel 17 hergestellt. ¹H-NMR (DMSO-d₆, ppm): 9,80 (s; breit), 1H), 9,39 (m , 1H), 9,20 (m, 1H), 8,54 (s, 1H), 7,62 (s, 1H), 7,30 (t, 1H), 6,92 - 6,80 (m, 3H), 5,00 (m, 1H), 3,49 (m, 2H), 3,18 (m, 2H), 2,38 (m, 2H), 2,17 (m, 2H).

### Beispiel 127: 5-(3-Fluor-phenyl)-7-piperidin-4-yl-7H-pyrrolo[2,3-d]pyrimidin-4-ylamin-dihydrochlorid

Wird ausgehend von 4-[4-Amino-5-(3-fluor-phenyl)-pyrrolo[2,3-d]pyrimidin-7-yl]-piperidin-1-carbonsäure-*tert*.-butylester (Beispiel 120) analog Beispiel 17 hergestellt. ¹H-NMR (DMSOd₆, ppm): 9,45 (m,1H), 9,32 (m, 1H), 8,58 (s, 1H), 7,78 (s, 1H), 7,55 (m, 1H), 7,38 -7,15 (m, 3H), 5,00 (m, 1H), 3,41 (m, 2H), 3,15 (m, 2H), 2,35 (m, 2H), 2,14 (m, 2H).

Analog Beispiel 16 werden ausgehend von 5-Phenyl-*7H*-pyrrolo[2,3-d]pyrimidin-4-yl-amin resp. 5-(4-Methoxy-phenyl)-*7H*-pyrrolo[2,3-d]pyrimidin-4-yl-amin resp. 5-(3-Methoxyphenyl)-*7H*-pyrrolo[2,3-d]pyrimidin-4-yl-amin und den entsprechend 1-substituierten 4-(Toluyl-4-sulfonyloxy)-piperidinen die Beispiele 128 - 142 hergestellt.

### Beispiel 128: 7-[1-(2-Morpholin-4-yl-ethyl)-piperidin-4-yl]-5-phenyl-7H-pyrrolo[2,3-d]pyrimidin-4-yl-amin

### Beispiel 129: 7-{1-[2-(2-Amino-ethoxy)-ethyl]-piperidin-4-yl]-5-phenyl-7H-pyrrolo[2,3-d]pyrimidin-4-yl-amin

### Beispiel 130: 7-[1-(2-Imidazol-1-yl-ethyl)-piperidin-4-yl]-5-phenyl-7H-pyrrolo[2,3-d]pyrimidin-4-yl-amin

### Beispiel 131: 7-[1-(2-Methoxy-ethyl)-piperidin-4-yl]-5-phenyl-7H-pyrrolo[2,3-d]pyrimidin-4-ylamin

### Beispiel 132: 2-[4-(4-Amino-5-phenyl-pyrrolo[2,3-d]pyrimidin-7-yl)-piperidin-1-yl]-ethanol

### Beispiel 133: 5-(4-Methoxy-phenyl)-7-[1-(2-morpholin-4-yl-ethyl)-piperidin-4-yl]-7H-pyrrolo-[2,3-d]pyrimidin-4-yl-amin

### Beispiel 134: 5-(3-Methoxy-phenyl)-7-[1-(2-morpholin-4-yl-ethyl)-piperidin-4-yl]-7H-pyrrolo-[2,3-d]pyrimidin-4-yl-amin

### Beispiel 135: 7-{1-[2-(2-Amino-ethoxy)-ethyl]-piperidin-4-yl}-5-(4-methoxy-phenyl)-7H pyrrolo[2,3-d]pyrimidin-4-yl-amin

### Beispiel 136: 7-{1-[2-(2-Amino-ethoxy)-ethyl]-piperidin-4-yl}-5-(3-methoxy-phenyl)-7H pyrrolo[2,3-d]pyrimidin-4-yl-amin

### Beispiel 137: 7-[1-(2-Imidazol-1-yl-ethyl)-piperidin-4-yl]-5-(4-methoxy-phenyl)-7H-pyrrolo-[2,3-d]pyrimidn-4-yl-amin

### Beispiel 138: 7-[1-(2-Imidazol-1-yl-ethyl)-piperidin-4-yl]-5-(3-methoxy-phenyl)-7H-pyrrolo-[2,3-d]pyrimidn-4-yl-amin

### Beispiel 139: 7-[1-(2-Methoxy-ethyl)-piperidin-4-yl]-5-(4-methoxy-phenyl)-7H-pyrrolo[2,3-d]-pyrimidin-4-yl-amin

### Beispiel 140: 7-[1-(2-Methoxy-ethyl)-piperidin-4-yl]-5-(3-methoxy-phenyl)-7H-pyrrolo[2,3-d]-pyrimidin-4-yl-amin

### Beispiel 141: 2-{4-[4-Amino-5-(4-methoxy-phenyl)-pyrrolo[2,3-d]pyrimidin-7-yl]-piperidin-1-yl}-ethanol

### Beispiel 142: 2-{4-[4-Amino-5-(3-methoxy-phenyl)-pyrrolo[2,3-d]pyrimidin-7-yl]-piperidin-1-yl}-ethanol

### Beispiel 143: 2-{4-[4-Amino-5-(3-methoxy-phenyl)-pyrrolo[2,3-d]pyrimidin-7-yl]-piperidin-1-yl}-ethanol

¹H-NMR (CDCl₃, ppm): 8,32 (s, 1H), 7,36 (t, 1 H), 7,09 - 6,90 (m, 4H), 5,20 (s, 2H), 4,75 (m, 1H), 3,85 (s, 3H), 3,68 (t, 2H), 3,09 (m, 2H), 2,61 (t, 2H), 2,35 (m, 2H), 2,19-1,90 (m, 4H)

### Beispiel 144: {4-[4-Amino-5-(3-methoxy-phenyl)-pyrrolo[2,3-d]pyrimidin-7-yl]-piperidin-1-yl}essigsäure-methylester

¹H-NMR (CDCl₃, ppm): 8,31 (s, 1H), 7,38 (t, 1H), 7,10 - 6,88 (m, 4H), 5,18 (s, 2H), 4,75 (m, 1H), 3,85 (s, 3H), 3,74 (s, 3H), 3,30 (s, 2H), 3,10 (m, 2H), 2,49 (m, 2H), 2,21 - 2,02 (m, 4H).

### Beispiel 145: 2-{4-[4-Amino-5-(3-methoxy-phenyl)-pyrrolo[2,3-d]pyrimidin-7-yl]-piperidin-1-yl}-acetamid

¹H-NMR (DMSO-d₆, ppm): 8,13 (s, 1H), 7,48 (s, 1H), 7,38 (t, 1H), 7,22 (s, 1H), 7,18 (s, 1H), 7,07 - 7,01 (m, 2H), 6,90 (m, 1H), 6,12 (s; breit), 2H), 4,61 (s, 1H), 3,82 (s, 3H), 3,35 (m, 2H), 2,98 (s, 2H), 2,31 (m, 2H), 2,16 (m, 2H), 1,88 (m, 2H).

### Beispiel 146: 2-{4-[4-Amino-5-(3-methoxy-phenyl)-pyrrolo[2,3-dipyrimidin-7-yl]-piperidin-1-yl}-N,N-dimethyl-acetamid

Smp: 214 -216° C.

### Beispiel 147: {4-[4-Amino-5-(3-methoxy-phenyl)-pyrrolo[2,3-d]pyrimidin-7-yl]-piperidin-1-yl}essigsäure

¹H-NMR (DMSO-d₆, ppm): 8,30 (s, 1H), 7,58 (s, 1H), 7,40 (t, 1H), 7,09 - 6,90 (m, 3H), 4,80 (m, 1H), 4,12 (s, 2H), 3,70 (s, 3H), 3,60 (m, 2H), 3,30 (m,2H), 2,50 (m, 2H), 2,18 (m, 2H).

Analog Beispiel 20 werden ausgehend von 5-(4-Benzyloxy-phenyl)-*7H*-pyrrolo[2,3-d]pyrimidin-4-yl-amin resp. 5-(3-Benzyloxy-phenyl)-*7H*-pyrrolo[2,3-d]pyrimidin-4-yl-amin und den entsprechend 1-substituierten 4-(Toluyl-4-sulfonyloxy)-piperidinen die Beispiele 148 - 157 hergestellt.

### Beispiel 148: 4-{4-Amino-7-[1-(2-hydroxy-ethyl)-piperidin-4-yl]-7H-pyrrolo[2,3-d]pyrimidin-5-yl}-phenol

### Beispiel 149: 3-{4-Amino-7-[1-(2-hydroxy-ethyl)-piperidin-4-yl]-7H-pyrrolo[2,3-d]pyrimidin-5-yl}-phenol

### Beispiel 150: 4-{4-Amino-7-[1-(2-morpholin-4-yl-ethyl)-piperidin-4-yl]-7H-pyrrolo[2,3-d]pyrimidin-5-yl}-phenol

### Beispiel 151: 3-{4-Amino-7-[1-(2-morpholin-4-yl-ethyl)-piperidin-4-yl]-7H-pyrrolo[2,3-d]pyrimidin-5-yl}-phenol

### Beispiel 152: 4-(4-Amino-7-{1-[2-(2-amino-ethoxy)-ethyl]-piperidin-4-yl}-7H-pyrrolo[2,3-d]-pyrimidin-5-yl)-phenol

### Beispiel 153: 3-(4-Amino-7-{1-[2-(2-amino-ethoxy)-ethyl]-piperidin-4-yl}-7H-pyrrolo[2,3-d]-pyrimidin-5-yl)-phenol

### Beispiel 154: 4-{4-Amino-7-[1-(2-methoxy-ethyl)-piperidin-4-yl]-7H-pyrrolo[2,3-d]pyrimidin-5-yl}-phenol

### Beispiel 155: 3-{4-Amino-7-[1-(2-methoxy-ethyl)-piperidin-4-yl]-7H-pyrrolo[2,3-d]pyrimidin-5-yl}-phenol

### Beispiel 156: 4-{4-Amino-7-[1-(2-imidazol-1-yl-ethyl)-piperidin-4-yl]-7H-pyrrolo[2,3-d]pyrimidin-5-yl}-phenol

### Beispiel 157: 3-{4-Amino-7-[1-(2-imidazol-1-yl-ethyl)-piperidin-4-yl]-7H-pyrrolo[2,3-d]pyrimidin-5-yl}-phenol

### Beispiel 158: {4-[4-Amino-5-(3-hydroxy-phenyl)-pyrrolo[2,3-d]pyrimidin-7-yl]-piperidin-1-yl)-essigsäure-methylester

### Beispiel 159: 2-{4-[4-Amino-5-(3-hydroxy-phenyl)-pyrrolo[2,3-d]pyrimidin-7-yl]-piperidin-1-yl}-N, N-dimethyl-acetamid

### Beispiel 160: 2-{4-[4-Amino-5-(3-hydroxy-phenyl)-pyrrolo[2,3-d]pyrimidin-7-yl]-piperidin-1-yl}acetamid

### Beispiel 161: {4-[4-Amino-5-(3-hydroxy-phenyl)-pyrrolo[2,3-dipyrimidin-7-yl]-piperidin-1-yl}essigsäure

### Beispiele A-B: Pharmazeutische Präparate

### Beispiel A: Tabletten, enthaltend ie 50 mg Wirkstoff:

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g der Kartoffelstärke vermischt und die Mischung mit einer ethanolischen Lösung der Gelatine befeuchtet und durch ein Sieb granuliert. Nach dem Trocknen mischt man den Rest der Kartoffelstärke, das Magnesiumstearat, den Talk und das Siliciumdioxid zu und presst das Gemisch zu Tabletten von je 145 mg Gewicht und 50 mg Wirkstoffgehalt, die gewünschtenfalls mit Teilkerben zur feineren Anpassung der Dosierung versehen sein können.

### Beispiel B: Lacktabletten, enthaltend je 100 mg Wirkstoff:

| Zusammensetzung (1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt. Das Gemisch wird mit einem Kleister, hergestellt aus 15 g Maisstärke und Wasser (unter Erwärmen), befeuchtet und granuliert. Das Granulat wird getrocknet und der Rest der Maisstärke, der Talk und das Calciumstearat werden mit dem Granulat vermischt. Das Gemisch wird zu Tabletten (Gewicht: je 280 mg) verpresst und diese werden mit einer Lösung der Hydroxypropylmethylcellulose und des Schellacks in Dichlormethan lackiert (Endgewicht der Lacktabletten: je 283 mg).

## Patentansprüche

1. Verbindungen der Formel I, 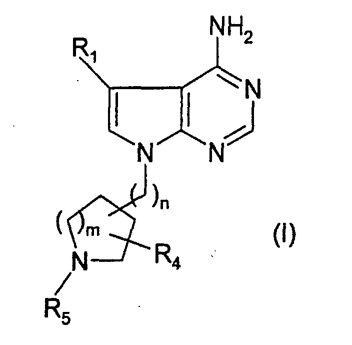 worin
R₁ für Phenyl steht, das unsubstituiert oder durch einen, zwei oder drei Substituenten aus der Gruppe bestehend aus Niederalkyl, Hydroxyniederalkyl, Phenyl, Niederalkoxy, Phenyl-niederalkoxy, C₁-C₃-Alkylendioxy, Cyano Hydroxy und Halogen substituiert ist;
R₄ für Wasserstoff, unsubstituiertes oder substituiertes Niederalkyl, Niederalkoxy, Niederalkytenoxyniederalkyl, Niederatkoxycarbonyl, N-Niederalkylaminocarbonyl, N,N-Diniederalkylaminocarbonyl, wobei die Substituenten aus der Gruppe bestehend aus Halogen, Hydroxy, Niederalkoxy Trifluromethyl, Morpholin-4-yl, Amino N-Niederalkylamino oder N,N-Diniederalkylamino ausgewählt sind;
R₅ für Wasserstoff oder unsubstitiertes oder substituiertes Niederalkyl, Niederalkylcarbonyl oder Niederalkoxycarbonyl wobei die Substituenten aus der Gruppe bestehend aus Halogen, Hydroxy, Niederalkoxy, Trifluromethyl, Morpholin-4-yl, Amino oder N-Niederalkylamino oder N-N-Diniederalkylamino ausgewählt sind;
m für 1 oder2 steht;
n für 0 oder 1 steht;
"Nieder" bezeichnet einen Rest bis und mit 7 Kohlenstoffatomen;
und Salze davon;
und
(*2R*/*4S*)-4-[4-Amino-5-(4-hydroxy-phenyl)-pyrrolo[2,3-d]pyrimidin-7-yl]-1-(3,3-dimethylbutyryl)-pyrrolidin-2-carbonsäure-ethylester,
(2S/4R)-2-(4-Amino-5-phenyl-pyrrolo[2,3-d]pyrimidin-7-ylmethyl)-4-hydroxy-pyrrolidin-1-carbonsäure-*tert*.-butylester,
(3R/5S)-5-(4-Amino-5-phenyl-pyrrolo[2,3-d]pyrimidin-7-ylmethyl)-pyrrolidin-3-ol,
(*2R*/*4R*)-2-(4-Amino-5-phenyl-pyrrolo[2,3-d]pyrimidin-7-ylmethyl)-4-hydroxy-pyrrolidin-1-carbonsäure-*tert*.-butylester,
(*3R*/*5R*)-5-(4-Amino-5-phenyl-pyrrolo[2,3-d]pyrimidin-7-ylmethyl)-pyrrolidin-3-ol,
7-[1-(2-lmidazol-1-yl-ethyl)-pyrrolidin-3-yl]-5-phenyl-7H-pyrrolo[2,3-d]pyrimidin-4-yl-amin, 4-(4-Amino-7-{1-[2-(2-hydroxy-ethylamino)-ethyl]-pyrrolidin-3-yl}-*7H*-pyrrolo[2,3-d]pyrimidin-5-yl)-phenol,
3-(4-Amino-7-{1-[2-(2-hydroxy-ethylamino)-ethyl]-pyrrolidin-3-yl}-*7H-*pyrrolo-[2,3-d]pyrimidin-5-yl)-phenol,
4-{4-Amino-7-[1-(2-imidazol-1-yl-ethyl)-pyrrolidin-3-yl]-7H-pyrrolo[2,3-d]pyrimidin-5-yl}phenol,
3-{4-Amino-7-[1-(2-imidazol-1-yl-ethyl}-pyrrolidin-3-yl]-*7H*-pyrrolo[2,3-d]pyrimidin-5-yl}phenol,
{3-[4-Amino-5-(3-benzyloxy-phenyl)-pyrrolo[2,3-d]pyrimidin-7-yl]-pyrrolidin-1-yl}essigsäure-methylester,
{3-[4-Amino-5-(3-hydroxy-phenyl)-pyrrolo[2,3-d]pyrimidin-7-yl]-pyrrolidin-1-yl}essigsäure-methylester,
2-{3-[4-Amino-5-(3-hydroxy-phenyl)-pyrrolo[2,3-d]pyrimidin-7-yl]-pyrrolidin-1-yl}-N,N-dimethyl-acetamid,
2-{3-[4-Amino-5-(3-hydroxy-phenyl)-pyrrolo[2,3-d]pyrimidin-7-yl]-pyrrolidin-1-yl}acetamid,
2-(2-{3-[4-Amino-5-(4-methoxy-phenyl)-pyrrolo[2,3-d]pyrimidin-7-yl]-pyrrolidin-1-yl}ethylamino)-ethanol,
2-(2-{3-[4-Amino-5-(3-methoxy-phenyl)-pyrrolo[2,3-d]pyrimidin-7-yl]-pyrrolidin-1-yl}ethylamino)-ethanol,
7-[1-(2-Imidazol-1-yl-ethyl)-pyrrolidin-3-yl]-5-(4-methoxy-phenyl)-*7H*-pyrrolo[2,3-d]-pyrimidin-4-yl-amin,
7-[1-[2-Imidazol-1-yl-ethyl)-pyrrolidin-3-yl]-5-(3-methoxy-phenyl)-*7H*-pyrrolo[2,3-d]-pyrimidin-4-yl-amin,
{3-[4-Amino-5-(3-methoxy-phenyl)-pyrrolo[2,3-d]pyrimidin-7-yl]-pyrrolidin-1-yl}essigsäure-methylester,
7-{1-[2-(2-Amino-ethoxy)-ethyl]-piperidin-4-yl}-5-phenyl-*7H*-pyrrolo[2,3-d]pyrimidin-4-ylamin,
7-[1-(2-Imidazol-1-yl-ethyl)-piperidin-4-yl]-5-phenyl-*7H*-pyrrolo[2,3-d]pyrimidin-4-yl-amin,
7-{1-[2-(2-Amino-ethoxy)-ethyl]-piperidin-4-yl}-5-(4-methoxy-phenyl)-*7H*-pyrrolo[2,3-d]-pyrimidin-4-yl-amin,
7-{1-[2-(2-Amino-ethoxy)-ethyl]-piperidin-4-yl}-5-(3-methoxy-phenyl)-*7H*-pyrrolo[2,3-d]-pyrimidin-4-yl-amin,
7-[1-(2-Imidazol-1-yl-ethyl)-piperidin-4-yl]-5-(4-methoxy-phenyl)-*7H*-pyrrolo[2,3-d]-pyrimidn-4-yl-amin,
7-[1-(2-lmidazol-1-yl-ethyl)-piperidin-4-yl]-5-(3-methoxy-phenyl)-*7H*-pyrrolo[2,3-d]-pyrimidn-4-yl-amin,
{4-[4-Amino-5-(3-methoxy-phenyl)-pyrrolo[2,3-d]pyrimidin-7-yl]-piperidin-1-yl}essigsäure-methylester,
2-{4-[4-Amino-5-(3-methoxy-phenyl)-pyrrolo[2,3-d]pyrimidin-7-yl]-piperidin-1-yl}acetamid,
2-{4-[4-Amino-5-(3-methoxy-phenyl)-pyrrolo[2,3-d]pyrimidin-7-yl]-piperidin-1-yl}-N,N-dimethyl-acetamid,
{4-[4-Amino-5-(3-methoxy-phenyl)-pyrrolo[2,3-d]pyrimidin-7-yl]-piperidin-1-yl}essigsäure,
4-(4-Amino-7-{1-[2-(2-amino-ethoxy)-ethyl]-piperidin-4-yl}-*7H*-pyrrolo[2,3-d]pyrimidin-5-yl)-phenol,
3-(4-Amino-7-{1-[2-(2-amino-ethoxy)-ethyl]-piperidin-4-yl}-*7H*-pyrrolo[2,3-d]pyrimidin-5-yl)-phenol,
4-{4-Amino-7-[1-(2-imidazol-1-yl-ethyl)-piperidin-4-yl]-*7H*-pyrrolo[2,3-d]pyrimidin-5-yl}phenol,
3-{4-Amino-7-[1-(2-imidazol-1-yl-ethyl)-piperidin-4-yl]*-7H*-pyrrolo[2,3-d]pyrimidin-5-yl}phenol,
{4-[4-Amino-5-(3-hydroxy-phenyl)-pyrrolo[2,3-d]pyrimidin-7-yl]-piperidin-1-yl}-essigsäuremethylester,
2-{4-[4-Amino-5-(3-hydroxy-phenyl)-pyrrolo[2,3-d]pyrimidin-7-yl]-piperidin-1-yl}-N,N-dimethyl-acetamid,
2-{4-[4-Amino-5-(3-hydroxy-phenyl)-pyrrolo[2,3-d]pyrimidin-7-yl]-piperidin-1-yl}-acetamid und {4-[4-Amino-5-(3-hydroxy-phenyl)-pyrrolo[2,3-d]pyrimidin-7-yl]-piperidin-1-yl}essigsäure.

2. Pharmazeutische Präparate enthaltend eine Verbindung gemäss Anspruch 1 und mindestens ein pharmazeutisch verwendbares Trägermaterial.

3. Eine Verbindung gemäss Anspruch 1 zur Anwendung in einem Verfahren zur therapeutischen Behandlung des tierischen oder menschlichen Körpers.

4. Eine Verbindung gemäss Anspruch 1 zur Verwendung bei der Behandlung von Krankheiten, die auf eine Hemmung der Aktivität der Protein-Tyrosin-Kinase pp60^{c-src} ansprechen.

5. Verwendung einer Verbindung gemäss Anspruch 1 zur Herstellung pharmazeutischer Präparate.

6. Verwendung einer Verbindung gemäss Anspruch 1 zur Herstellung pharmazeutischer Präparate für die Behandlung von Krankheiten, die auf eine Hemmung der Aktivität der Protein-Tyrosin-Kinase pp60^{c-src} ansprechen.

7. Verfahren zur Herstellung einer Verbindung der Formel I gemäss Anspruch 1, **dadurch gekennzeichnet, dass** man
(a) eine Verbindung der Formel II 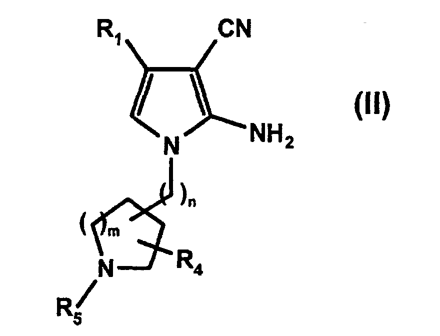 einer Ringschlussreaktion unter Aufbau des Pyrimidinrings unterwirft, oder
(b) eine Verbindung der Formel III 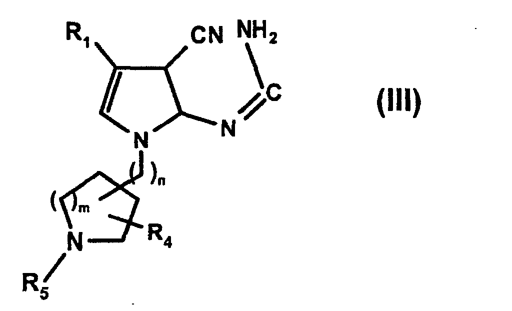 einer Ringschlussreaktion unter Aufbau des Pyrimidinrings unterw
irft, oder
(c) eine Verbindung der Formel IV 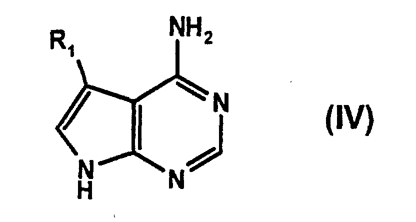 mit einer Verbindung der Formel V 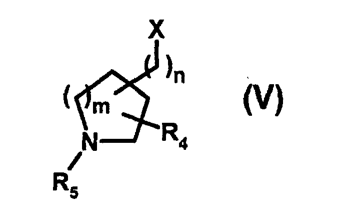 worin X für eine Abgangsgruppe steht, umsetzt, oder
(d) eine Verbindung der Formel VI 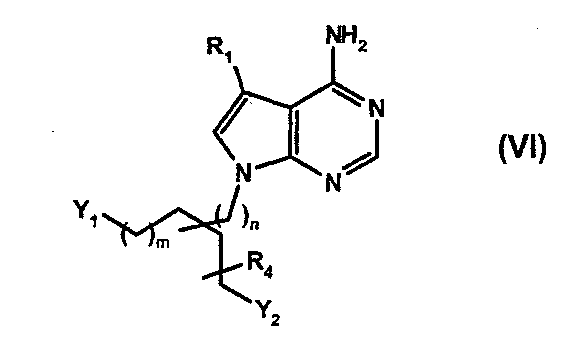
worin Y₁ und Y₂ für geeignete Abgangsgruppen stehen, mit einer Verbindung der Formel VII
**R**_{**5**}**―NH**_{**2**} **(VII)**
umsetzt,
und, wenn erwünscht, eine Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

## Claims

1. Compounds of formula I 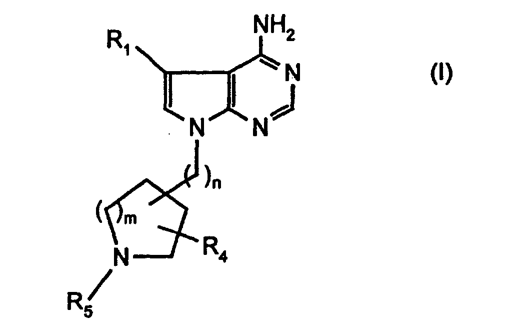 wherein
R₁ is phenyl which is unsubstituted or substituted by one, two or three substituents from the group consisting of lower alkyl, hydroxy-lower alkyl, phenyl, lower alkoxy, phenyl-lower alkoxy, C₁-C₃alkylenedioxy, cyano, hydroxy and halogen;
R₄ is hydrogen, unsubstituted or substituted lower alkyl, lower alkoxy, lower alkyleneoxy-lower alkyl, lower alkoxycarbonyl, N-lower alkylaminocarbonyl, N,N-di-lower alkylaminocarbonyl, the substituents being selected from the group consisting of halogen, hydroxy, lower alkoxy, trifluoromethyl, morpholin-4-yl, amino, N-lower alkylamino and N,N-di-lower alkylamino;
R₅ is hydrogen or unsubstituted or substituted lower alkyl, lower alkylcarbonyl or lower alkoxycarbonyl, the substituents being selected from the group consisting of halogen, hydroxy, lower alkoxy, trifluoromethyl, morpholin-4-yl, amino, N-lower alkylamino and
N,N-di-lower alkylamino;
m is 1 or 2;
n is 0 or 1;
"lower" denoting a radical having up to and including 7 carbon atoms;
and salts thereof;
and:
(*2R*/*4S*)-4-[4-amino-5-(4-hydroxy-phenyl)-pyrrolo[2,3-d]pyrimidin-7-yl]-1-(3,3-dimethylbutyryl)-pyrrolidine-2-carboxylic acid ethyl ester,
(2S/4R)-2-(4-amino-5-phenyl-pyrrolo[2,3-d]pyrimidin-7-ylmethyl)-4-hydroxy-pyrrolidine-1-carboxylic acid *tert*-butyl ester,
(3R/5S)-5-(4-amino-5-phenyl-pyrrolo[2,3-d]pyrimidin-7-ylmethyl)-pyrrolidin-3-ol,
(*2R*/*4R*)-2-(4-amino-5-phenyl-pyrrolo[2,3-d]pyrimidin-7-ylmethyl)-4-hydroxy-pyrrolidine-1-carboxylic acid *tert*-butyl ester,
(*3R*/*5R*)-5-(4-amino-5-phenyl-pyrrolo[2,3-d]pyrimidin-7-ylmethyl)-pyrrolidin-3-ol,
7-[1-(2-imidazol-1-yl-ethyl)-pyrrolidin-3-yl]-5-phenyl-*7H*-pyrrolo[2,3-d]pyrimidin-4-yl-amine,
4-(4-amino-7-{1-[2-(2-hydroxy-ethylamino)-ethyl]-pyrrolidin-3-yl}-*7H*-pyrrolo[2,3-d]pyrimidin-5-yl)-phenol,
3-(4-ammo-7-{1-{2-(2-hydroxy-ethylamino)-ethyl]-pyrrolidin-3-yl}-*7H*-pyrrolo[2,3-d]pyrimidin-5-yl)-phenol,
4-{4-amino-7-[1-(2-imidazot-1-yl-ethyl)-pyrrolidin-3-yl]-*7H*-pyrrolo[2,3-d]pyrimidin-5-yl}-phenol,
3-{4-amino-7-[1-(2-imidazol-1-yl-ethyl)-pyrrolidin-3-yl]-*7H*-pyrrolo[2,3-d]pyrimidin-5-yl}-phenol,
{3-[4-amino-5-(3-benzyloxy-phenyl)-pyrrolo[2,3-d]pyrimidin-7-yl]-pyrrolidin-1-yl}-acetic acid methyl ester,
{3-[4-amino-5-(3-hydroxy-phenyl)-pyrrolo[2,3-d]pyrimidin-7-yl]-pyrrolidin-1-yl}-acetic acid methyl ester,
2-{3-[4-amino-5-(3-hydroxy-phenyl)-pyrrolo[2,3-d]pyrimidin-7-yl]-pyrrolidin-1-yl}-N,N-dimethyl-acetamide,
2-{3-[4-amino-5-(3-hydroxy-phenyl)-pyrrolo[2,3-d]pyrimidin-7-yl]-pyrrolidin-1-yl}-acetamide,
2-(2-{3-[4-amino-5-(4-methoxy-phenyl)-pyrrolo[2,3-d]pyrimidin-7-yl]-pyrrolidin-1-yl}-ethylamino)-ethanol,
2-(2-{3-[4-amino-5-(3-methoxy-phenyl)-pyrrolo[2,3-d]pyrimidin-7-yl]-pyrrolidin-1-yl}-ethylamino)-ethanol,
7-[1-(2-imidazol-1-yl-ethyl)-pyrrolidin-3-yl]-5-(4-methoxy-phenyl)*-7H*-pyrrolo[2,3-d]pyrimidin-4-yl-amine,
7-[1-(2-imidazol-1-yl-ethyl)-pyrrolidin-3-yl]-5-(3-methoxy-phenyl)*-7H*-pyrrolo[2,3-d]pyrimidin-4-yl-amine,
{3-[4-amino-5-(3-methoxy-phenyl)-pyrrolo[2,3-d]pyrimidin-7-yl]-pyrrolidin-1-yl}-acetic acid methyl ester,
7-{1-[2-(2-amino-ethoxy}-ethyl]-piperidin-4-yl}-5-phenyl-*7H*-pyrrolo[2,3-d]pyrimidin-4-yl-amine,
7-[1-(2-imidazol-1-yl-ethyl)-piperidin-4-yl]-5-phenyl-*7H*-pyrrolo[2,3-d]pyrimidin-4-yl-amine,
7-{1-[2-(2-amino-ethoxy)-ethyl]-piperidin-4-yl}-5-(4-methoxy-phenyl)-*7H*-pyrrolo[2,3-d]-pyrimidin-4-yl-amine,
7-{1-[2-(2-amino-ethoxy}-ethyl]-piperidin-4-yl}-5-(3-methoxy-phenyl)-*7H*-pyrrolo[2,3-d]-pyrimidin-4-yl-amine,
7-[1-(2-imidazol-1-yl-ethyl)-piperidin-4-yl]-5-(4-methoxy-phenyl)-*7H*-pyrrolo[2,3-d]pyrimidin-4-yl-amine,
7-[1-(2-imidazol-1-yl-ethyl)-piperidin-4-yl]-5-(3-methoxy-phenyl)-*7H*-pyrrolo[2,3-d]pyrimidin-4-yl-amine,
{4-[4-amino-5-(3-methoxy-phenyl)-pyrrolo[2,3-d]pyrimidin-7-yl]-piperidin-1-yl}-acetic acid methyl ester,
2-{4-[4-amino-5-(3-methoxy-phenyl)-pyrrolo[2,3-d]pyrimidin-7-yl]-piperidin-1-yl}-acetamide,
2-{4-[4-amino-5-(3-methoxy-phenyl)-pyrrolo[2,3-d]pyrimidin-7-yl]-piperidin-1-yl}-N,N-dimethyl-acetamide,
{4-[4-amino-5-(3-methoxy-phenyl)-pyrrolo[2,3-d]pyrimidin-7-yl]-piperidin-1-yl}-acetic acid,
4-(4-amino-7-{1-[2-(2-amino-ethoxy)-ethyl]-piperidin-4-yl}-*7H*-pyrrolo[2,3-d]pyrimidin-5-yl)-phenol,
3-(4-amino-7-{1-[2-(2-amino-ethoxy)-ethyl]-piperidin-4-yl}-*7H*-pyrrolo[2,3-d]pyrimidin-5-yl)-phenol,
4-{4-amino-7-[1-(2-imidazol-1-yl-ethyl)-piperidin-4-yl]-*7H*-pyrrolo[2,3-d]pyrimidin-5-yl}-phenol,
3-{4-amino-7-[1-(2-imidazol-1-yl-ethyl)-piperidin-4-yl]-*7H*-pyrrolo[2,3-d]pyrimidin-5-yl}-phenol,
{4-[4-amino-5-(3-hydroxy-phenyl)-pyrrolo[2,3-d]pyrimidin-7-yl]-piperidin-1-yl}-aceticacid methyl ester,
2-{4-[4-amino-5-(3-hydroxy-phenyl)-pyrrolo[2,3-d]pyrimidin-7-yl]-plperidin-1-yl}-N,N-dimethyl-acetamide,
2-{4-[4-amino-5-(3-hydroxy-phenyl)-pyrrolo[2,3-d]pyrimidin-7-yl]-piperidin-1-yl}-acetamide and {4-[4-amino-5-(3-hydroxy-phenyl)-pyrrolo[2,3-d]pyrimidin-7-yl]-piperidin-1-yl}-acetic acid.

2. Pharmaceutical compositions comprising a compound according to claim 1 and at least one pharmaceutically acceptable carrier.

3. A compound according to claim 1 for use in a method for the therapeutic treatment of the animal or human body.

4. A compound according to claim 1 for use in the treatment of diseases that are responsive to inhibition of the activity of protein tyrosine kinase pp60^{c-src}.

5. Use of a compound according to claim 1 in the preparation of pharmaceutical compositions.

6. Use of a compound according to claim 1 in the preparation of pharmaceutical compositions for the treatment of diseases that are responsive to inhibition of the activity of protein tyrosine kinase pp60^{c-src}.

7. Process for the preparation of a compound of formula I according to claim 1, which process comprises
(a) subjecting a compound of formula II 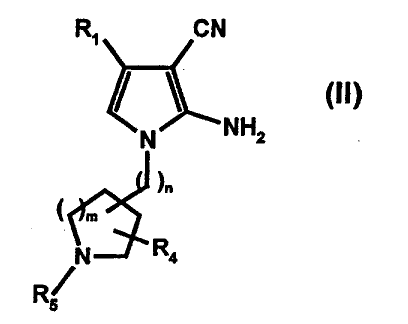 to a ring closure reaction with synthesis of the pyrimidine ring, or
(b) subjecting a compound of formula III 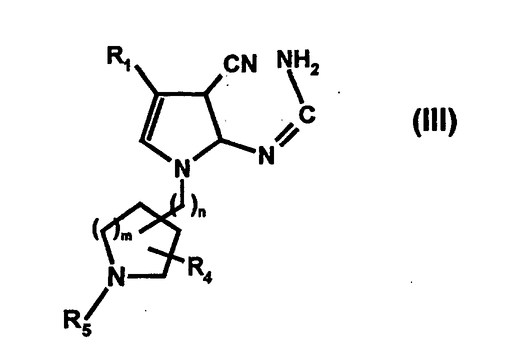 to a ring closure reaction with synthesis of the pyrimidine ring, or
(c) reacting a compound of formula IV 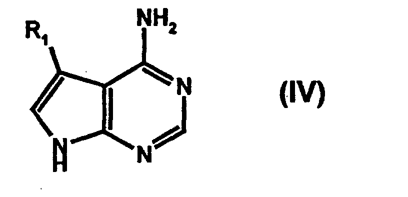 with a compound of formula V 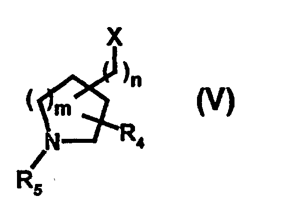 wherein X is a leaving group; or
(d) reacting a compound of formula VI 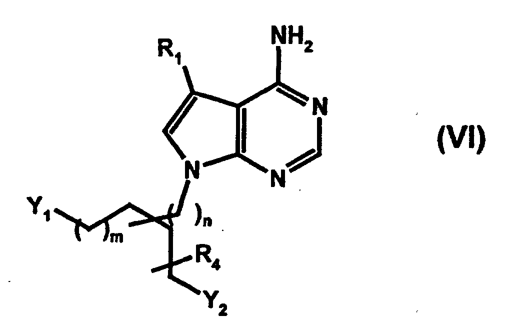
wherein Y₁ and Y₂ are suitable leaving groups with a compound of formula VII
**R**_{**5**}**―NH**_{**2**} **(VII)**
and, if desired, converting a compound of formula I into a different compound of formula I, and/or, if desired, converting a resulting salt into the free compound or into a different salt, and/or, if desired, converting a resulting free compound of formula I having salt-forming properties into a salt.

## Revendications

1. Composés de formule I, 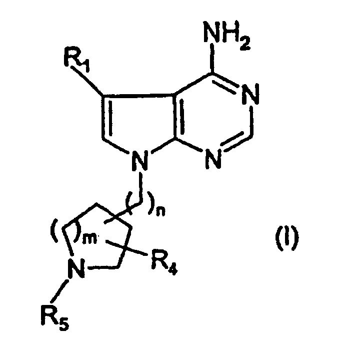 dans laquelle
R₁ est un groupe phényle, non substitué ou substitué par un, deux ou trois substituants choisis parmi le groupe composé d'un alkyle inférieur, d'un hydroxy-(alkyle inférieur), d'un phényle, d'un alcoxy inférieur, d'un phényl-alcoxy inférieur, d'un alkylène(en C₁-C₃)-dioxy, d'un cyano, d'un hydroxy et d'un halogène ;
R₄ est l'hydrogène, un alkyle inférieur, un alcoxy inférieur, un (alkylène inférieur)-oxy(alkyle inférieur), un (alcoxy inférieur)-carbonyle, un N-(alkyle inférieur)-aminocarbonyle, un N,N-di(alkyle inférieur)-aminocarbonyle non substitué ou substitué, les substituants étant choisis parmi le groupe composé d'un halogène, d'un hydroxy, d'un alcoxy inférieur, d'un trifluorométhyle, d'un morpholin-4-yle, d'un amino, d'un N-(alkyle inférieur)-amino ou d'un N,N-di(alkyle inférieur)-amine ;
R₅ est l'hydrogène ou un alkyle inférieur, un (alkyle inférieur)-carbonyle ou un (alcoxy inférieur)-carbonyle non substitué ou substitué, les substituants étant choisis parmi le groupe composé d'un halogène, d'un hydroxy, d'un alcoxy inférieur, d'un trifluorométhyle, d'un morpholin-4-yle, d'un amino, d'un N-(alkyle inférieur)-amino ou d'un N,N-di(alkyle inférieur)-amine ;
m vaut 1 ou 2 ;
n vaut 0 ou 1 ;
"inférieur" désigne un radical comportant jusqu'à 7 atomes de carbone ;
et leurs sels ;
et
l'éthylester d'acide (2R/4S)-4-[4-amino-5-(4-hydroxy-phényl)-pyrrolo[2,3-d]pyrimidin-7-yl]-1-(3,3-diméthyl-butyryl)-pyrrolidine-2-carboxylique,
le tert.-butylester de l'acide (2S/4R)-2-(4-amino-5-phényl-pyrrolo[2,3-d]pyrimidin-7-ylméthyl)-4-hydroxy-pyrrolidine-1-carboxylique,
le (3R/5S)-5-(4-amino-5-phényl-pyrrolo[2,3-d]pyrimidin-7-ylméthyl)-pyrrolidin-3-ol,
le tert.-butylester de l'acide (2R/4R)-2-(4-amino-5-phényl-pyrrolo[2,3-d]pyrimidin-7-ylméthyl)-4-hydroxy-pyrrolidine-1-carboxylique,
le (3R/5R)-5-(4-amino-5-phényl-pyrrolo[2,3-d]pyrrimidin-7-ylméthyl)-pyrrolidin-3-ol,
la 7-[1-(2-imidazol-1-yl-éthyl)-pyrrolidin-3-yl]-5-phényl-7H-pyrrolo[2,3-d]pyrimidin-4-yl-amine,
le 4-(4-amino-7-{1-[2-(2-hydroxy-éthylamino)-éthyl]-pyrrolidin-3-yl}-7H-pyrrolo[2,3-d]pyrimidin-5-yl)-phénol,
le 3-(4-amino-7- {1-[2-(2-hydroxy-éthylamino)-éthyl]-pyrrolidin-3-yl}-7H-pyrrolo[2,3-d]pyrimidin-5-yl)-phénol,
le 4-{4-amino-7-[1-(2-imidazol-1-yl-éthyl)-pyrrolidin-3-yl]-7H-pyrrolo[2,3-d]pyrimidin-5-yl}-phénol,
le 3-{4-amino-7-[1-(2-imidazol-1-yl-éthyl)-pyrrolidin-3-yl]-7H-pyrrolo[2,3-d]pyrimidin-5-yl}-phénol,
le méthylester de l'acide {3-[4-amino-5-(3-benzyloxy-phényl)-pyrrolo[2,3-d]pyrimidin-7-yl]-pyrrolidin-1-yl}acétique,
le méthylester de l'acide {3-[4-amino-5-(3-hydroxy-phényl)-pyrrolo[2,3-d]pyrimidin-7-yl]-pyrrolidin-1-yl}acétique,
le 2-{3-[4-amino-5-(3-hydroxy-phényl)-pyrrolo[2,3-d]pyrimidin-7-yl]-pyrrolidin-1-yl}-N,N-diméthylacétamide,
le 2-{3-[4-amino-5-(3-hydroxy-phényl)-pyrrolo[2,3-d]pyrimidin-7-yl]-pyrrolidin-1-yl}-acétamide,
le 2-(2-{3-[4-amino-5-(4-méthoxy-phényl)-pyrrolo[2,3-d]pyrimidin-7-yl]pyrrolidin-1-yl}éthylamino)-éthanol,
le 2-(2-{3-[4-amino-5-(3-méthoxy-phényl)-pyrrolo[2,3-d]pyrimidin-7-yl]pyrrolidin-1-yl}éthylamino)-éthanol,
la 7-[1-(2-imidazol-1-yl-éthyl)-pyrrolidin-3-yl]-5-(4-méthoxy-phényl)-7H-pyrrolo[2,3-d]pyrimidin-4-yl-amine,
la 7-[1-(2-imidazol-1-yl-éthyl)-pyrrolidin-3-yl]-5-(3-méthoxy-phényl)-7H-pyrrolo[2,3-d]pyrimidin-4-yl-amine,
le méthylester de l'acide {3-[4-amino-5-(3-méthoxy-phényl)-pyrrolo[2,3-d]pyrimidin-7-yl]-pyrrolidin-1-yl}acétique,
la 7- 1-[2-(2-amino-éthoxy)-éthyl]-pipéridin-4-yl}-5-phényl-7H-pyrrolo[2,3-d]pyrimidin-4-yl-amine,
la 7-[1-(2-imidazol-1-yl-éthyl)-pipéridin-4-yl]-5-phényl-7H-pyrrolo[2,3-d]pyrimidin-4-yl-amine,
la 7-{1-[2-(2-amino-éthoxy)-éthyl]-pipéridin-4-yl}-5-(4-méthoxy-phényl)-7H-pyrrolo[2,3-d]pyrimidin-4-yl-amine,
la 7-{1-[2-(2-amino-éthoxy)-éthyl]-pipéridin-4-yl}-5-(3-méthoxy-phényl)-7H-pyrrolo[2,3-d]-pyrimidin-4-yl-amine,
la 7-[1-(2-imidazol-1-yl-éthyl)-pipéridin-4-yl]-5-(4-méthoxy-phényl)-7H-pyrrolo[2,3-d]-pyrimidin-4-yl-amine,
la 7-[1-(2-imidazol-1-yl-éthyl)-pipéridin-4-yl]-5-(3-méthoxy-phényl)-7H-pyrrolo[2,3-d]-pyrimidin-4-yl-amine,
le méthylester de l'acide {4-[4-amino-5-(3-méthoxy-phényl)-pyrrolo[2,3-d]pyrimidin-7-yl]-pipéridin-1-yl}acétique,
le 2-{4-[4-amino-5-(3-méthoxy-phényl)-pyrrolo[2,3-d]pyrimidin-7-yl]-pipéridin-1-yl}acétamide,
le 2- {4-[4-amino-5-(3-méthoxy-phényl)-pyrrolo[2,3-d]pyrimidin-7-yl]-pipéridin-1-yl}-N,N-diméthylacétamide,
l'acide {4-[4-amino-5-(3-méthoxy-phényl)-pyrrolo[2,3-d]pyrimidin-7-yl]-pipéridin- 1-yl} acétique,
le 4-(4-amino-7-{1-[2-(2-amino-éthoxy)-éthyl]-pipéridin-4-yl} -7H-pyrrolo[2,3-d]pyrimidin-5-yl)-phénol,
le 3-(4-amine-7-{1-[2-(2-amino-éthoxy)-éthyl]-pipéridin-4-yl} -7H-pyrrolo[2,3-d]pyrimidin-5-yl)-phénol,
le 4-{4-amino-7-[1-(2-imidazol-1-yl-éthyl)-pipéridin-4-yl]-7H-pyrrolo[2,3-d]pyrimidin-5-yl}-phénol,
le 3-{4-amino-7-[1-(2-imidazol-1-yl-éthyl)-pipéridin-4-yl]-7H-pyrrolo[2,3-d]pyrimidin-5-yl}-phénol,
le méthylester de l'acide {4-[4-amino-5-(3-hydroxy-phényl)-pyrrolo[2,3-d]pyrimidin-7-yl]-pipéridin-1-yl}acétique,
le 2- {4-[4-amino-5-(3-hydroxy-phényl)-pyrrolo[2,3-d]pyrimidin-7-yl]-pipéridin-1-yl}-N,N-diméthylacétamide,
le 2-{4-[4-amino-5-(3-hydroxy-phényl)-pyrrolo[2,3-d]pyrimidin-7-yl]-pipéridin-1-yl}-acétamide et
l'acide {4-[4-amino-5-(3-hydroxy-phényl)-pyrrolo[2,3-d]pyrimidin-7-yl]-pipéridin-1-yl}-acétique.

2. Préparations pharmaceutiques contenant un composé selon la revendication 1 et au moins un matériau véhiculeur pouvant être utilisé en pharmacie.

3. Composé selon la revendication 1, destiné à être utilisé dans un procédé de traitement thérapeutique du corps animal ou humain.

4. Composé selon la revendication 1, destiné à être utilisé dans le traitement de maladies qui réagissent à une inhibition de l'activité de la protéine-tyrosine kinase pp60^{c-src}.

5. Utilisation d'un composé selon la revendication 1 pour fabriquer des préparations pharmaceutiques.

6. Utilisation d'un composé selon la revendication 1 pour fabriquer des préparations pharmaceutiques destinées au traitement de maladies qui réagissent à une inhibition de l'activité de la protéine-tyrosine kinase pp60^{c-src}.

7. Procédé de préparation d'un composé de formule I selon la revendication 1, **caractérisé en ce que**
(a) on soumet un composé de formule II 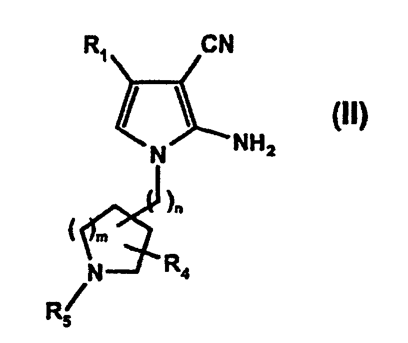 à une réaction de cyclisation, en formant le cycle pyrimidine, ou
(b) on soumet un composé de formule III 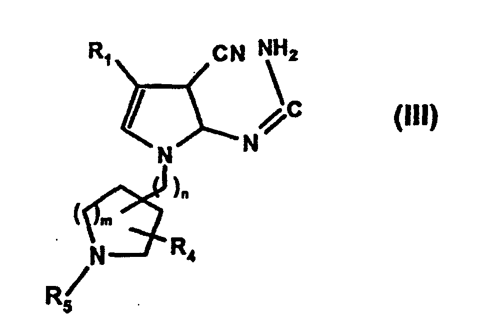 à une réaction de cyclisation, en formant le cycle pyrimidine, ou
(c) on fait réagir un composé de formule IV 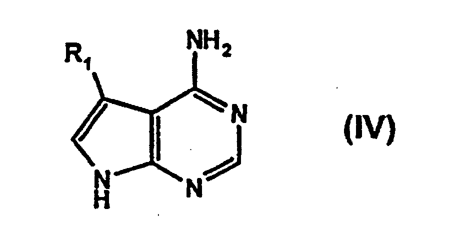 avec un composé de formule V 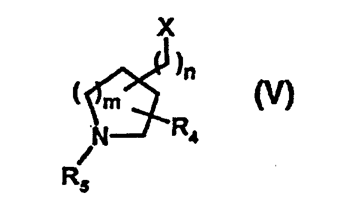 dans laquelle X est un groupe partant, ou
(d) on fait réagir un composé de formule VI 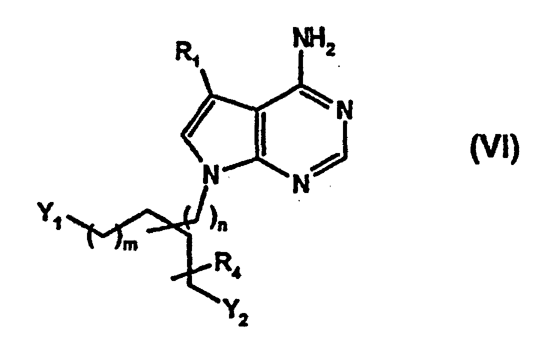
dans laquelle Y₁ et Y₂ sont des groupes partants appropriés, avec un composé de formule VII
**R**_{**5**}**―NH**_{**2**} **(VII)**
et, quand cela est souhaité, on convertit un composé de formule I en un autre composé de formule I, et/ou, quand cela est souhaité, on convertit un sel obtenu en forme libre du composé ou en un autre sel, et/ou, quand cela est souhaité, on convertit un composé libre obtenu de formule I présentant des propriétés salifiables en un sel.
